# EUROPEAN PATENT SPECIFICATION

(11) **EP 0 682 699 B1**
(45) Date of publication and mention of the grant of the patent: **07.05.2003**
(21) Application number: 94909526.9
(22) Date of filing: 10.02.1994
(51) Int. Cl.: C12N 9/22, C12N 15/55, C12N 15/74, C12N 15/62

(54) **FUNCTIONAL DOMAINS IN $i(FLAVOBACTERIUM OKEANOKOITES) (FOKI) RESTRICTION ENDONUCLEASE**
FUNKTIONELLE DOMÄNEN DER RESTRIKTIONSENDONUKLEASEN AUS -i(FLAVOBAKTERIUM OKEANOKOITES)(FOKI)
DOMAINES FONCTIONNELS DANS L'ENDONUCLEASE DE RESTRICTION D'OKEANOKOITES FLAVOBACTERIUM

(30) Priority: 12.02.1993 US 17493
(43) Date of publication of application: 22.11.1995
(73) Proprietor: THE JOHNS-HOPKINS UNIVERSITY, Baltimore, MD 21218 (US)
(72) Inventor: CHANDRASEGARAN, Srinivasan, Baltimore, MD 21218 (US)
(74) Representative: Tombling, Adrian George
(86) International application number: US9401201
(87) International publication number: WO94018313

(56) References cited:
- WO-A-92/22642
- PROC. NATL ACAD. SCI., vol. 91, no. 3, 1 February 1994, pages 883-887, XP002020280 KIM ET AL.: "Chimeric restriction endonuclease"
- PROC. NATL ACAD. SCI., vol. 90, no. 7, 1 April 1993, pages 2764-2768, XP002020281 LI ET AL.: "Alteration of the cleavage distance of FokI restriction endonuclease by insertion mutagenesis"
- GENE, vol. 54, no. 2-3, 1987, pages 229-238, XP002020282 VERMERSCH ET AL.: "The use of a selectable FokI cassette in DNA replacement mutagenesis of the R388 dihydrofolate reductase gene"
- Proc. Natl. Acad. Sci. USA, Volume 89, issued May 1992, L. LI et al., "Functional Domains in FokI Restriction Endonuclease", pages 4275-4279, see entire document.
- Nucl. Acids. Res., Volume 20, No. 16, issued 25 August 1992, K. KITA et al., "Cloning and Sequence Analysis of the StsI Restriction-Modification Gene: Presence of Homology to FokI Restriction-Modification Enzymes", pages 4167-4172, especially page 4167, column 2.
- Nucl. Acids. Res., Volume 19, No. 5, issued 11 March 1991, H. BOCKLAGE et al., "Cloning and Characterization of the MboII Restriction-Modification System", pages 1007-1013, see especially page 1007.
- J. Biol. Chem., Volume 264, No. 10, issued 05 April 1989, K. KITA et al., "The FokI Restriction-Modification System", pages 5751-5756.
- Gene, Volume 80, issued 1989, M.C. LOONEY et al., "Nucleotide Sequence of the FokI Restriction-Modification System; Separate Strand-Specificity Domains in the Methyltransferase", pages 193-208.

## Description

### BACKGROUND OF THE INVENTION

### 1. Field of the Invention:

The present invention relates to the *Fok*I restriction endonuclease system. In particular, the present invention relates to DNA segments encoding the separate functional domains of this restriction endonuclease system.

The present invention also relates to the construction of two insertion mutants of *Fok*I endonuclease.

### 2. Background Information:

Type II endonucleases and modification methylases are bacterial enzymes that recognize specific sequences in duplex DNA. The endonuclease cleaves the DNA while the methylases methylates adenine or cytosine residues so as to protect the host-genome against cleavage [Type II restriction and modification enzymes. In Nucleases (Eds. Modrich and Roberts) Cold Spring Harbor Laboratory, New York, pp. 109-154, 1982]. These restriction-modification (R-M) systems function to protect cells from infection by phage and plasmid molecules that would otherwise destroy them.

As many as 2500 restriction enzymes with over 200 specificities have been detected and purified (Wilson and Murray, Annu. Rev. Genet. 25:585-627, 1991). The recognition sites of most of these enzymes are 4-6 base pairs long. The small size of the recognition sites is beneficial as the phage genomes are usually small and these small recognition sites occur more frequently in the phage.

Eighty different R-M systems belonging to the Type IIS class with over 35 specificities have been identified. This class is unique in that the cleavage site of the enzyme is separate from the recognition sequence. Usually the distance between the recognition site and the cleavage site is quite precise (Szybalski et al., Gene, 100:13-26, 1991). Among all these enzymes, the *Fok*I restriction endonuclease is the most well characterized member of the Type IIS class. The *Fok*I endonuclease (*RFokI*) recognizes asymmetric pentanucleotides in double-stranded DNA, 5' GGATG-3' (SEQ ID NO: 1) in one strand and 3'-CCTAC-5' (SEQ ID NO: 2) in the other, and introduces staggered cleavages at sites away from the recognition site (Sugisaki et al., Gene 16:73-78; 1981). In contrast, the *Fok*I methylase (*MFokI*) modifies DNA thereby rendering the DNA resistant to digestion by *Fok*I endonuclease. The *Fok*I restriction and modification genes have been cloned and their nucleotide sequences deduced (Kita et al., J. of Biol. Chem., 264:575-5756, 1989). Nevertheless, the domain structure of the *Fok*I restriction endonuclease remains unknown, although a three domain structure has been suggested (Wilson and Murray, Annu. Rev. Genet. 25:585-627, 1991).

### SUMMARY OF THE INVENTION

Accordingly, it is an object of the present invention to provide hybrid restriction enzymes which are useful for mapping and sequencing.

Various other objects and advantages of the present invention will become obvious from the drawings and the following description of the invention.

In one embodiment, the present invention relates to a DNA segment encoding the recognition domain of a Type IIS endonuclease which contains the sequence-specific recognition activity of the Type IIS endonuclease or a DNA segment encoding the catalytic domain of a Type IIS endonuclease which contains the cleavage activity of the Type IIS endonuclease.

In another embodiment, the present invention relates to an isolated protein consisting essentially of the N-terminus or recognition domain of the *Fok*I restriction endonuclease which protein has the sequence-specific recognition activity of the endonuclease or an isolated protein consisting essentially of the C-terminus or catalytic domain of the *Fok*I restriction endonuclease which protein has the nuclease activity of the endonuclease.

In one embodiment, the present invention relates to a DNA construct comprising a first DNA segment encoding the catalytic domain of a Type IIS endonuclease which contains the cleavage activity of the Type IIS endonuclease; a second DNA segment encoding a sequence-specific recognition domain other than the recognition domain of the Type IIS endonuclease; a third DNA segment comprising one or more codons, wherein the third DNA segment is inserted between the first DNA segment and the second DNA segment; and a vector. Preferably, the third segment contains four or seven codons.

In another embodiment, the present invention relates to a procaryotic cell comprising a first DNA segment encoding the catalytic domain of a Type IIS endonuclease which contains the cleavage activity of the Type IIS endonuclease; a second DNA segment encoding a sequence-specific recognition domain other than the recognition domain of the Type IIS endonuclease; a third DNA segment comprising one or more codons, wherein the third DNA segment is inserted between the first DNA segment and the second DNA segment; and a vector. The first DNA segment and the second DNA segment are operably linked to the vector so that a single protein is produced.

### BRIEF DESCRIPTION OF THE DRAWINGS

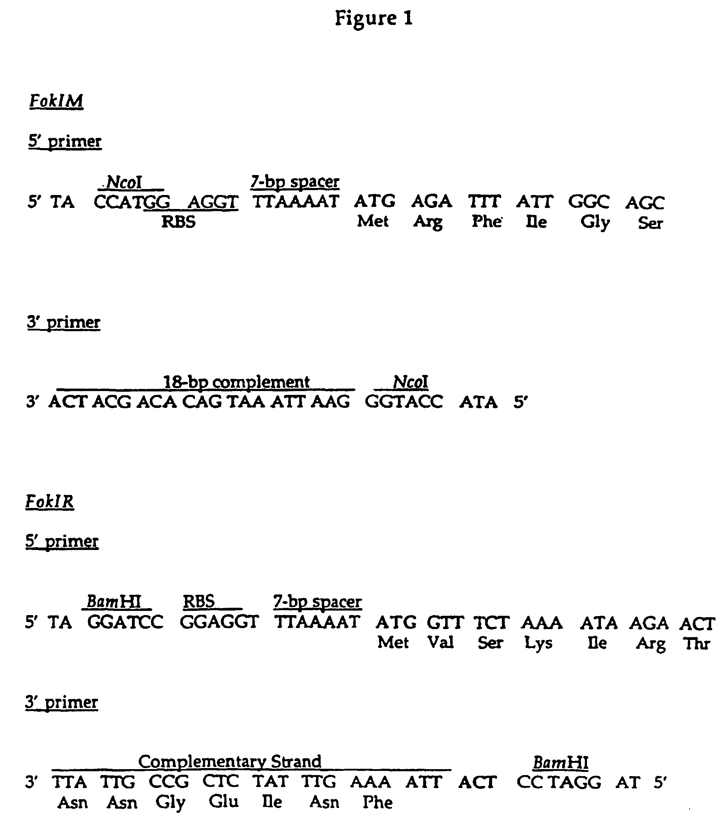

FIGURE 1 shows sequences of the 5' and 3' primers used to introduce new translation signals into *fokIM* and *fokIR* genes during PCR amplification. (SEQ ID NOs: 3-9). SD represents Shine-Dalgarno consensus RBS for *Escherichia coli* (*E. coli*) and 7-bp spacer separates the RBS from the ATG start condon. The *fokIM* primers are flanked by *NcoI* sites. The *fokIR* primers are flanked by *BamHI* sites. Start and stop codons are shown in bold letters. The 18-bp complement sequence is complementary to the sequence immediately following the stop codon of *MfokI* gene.

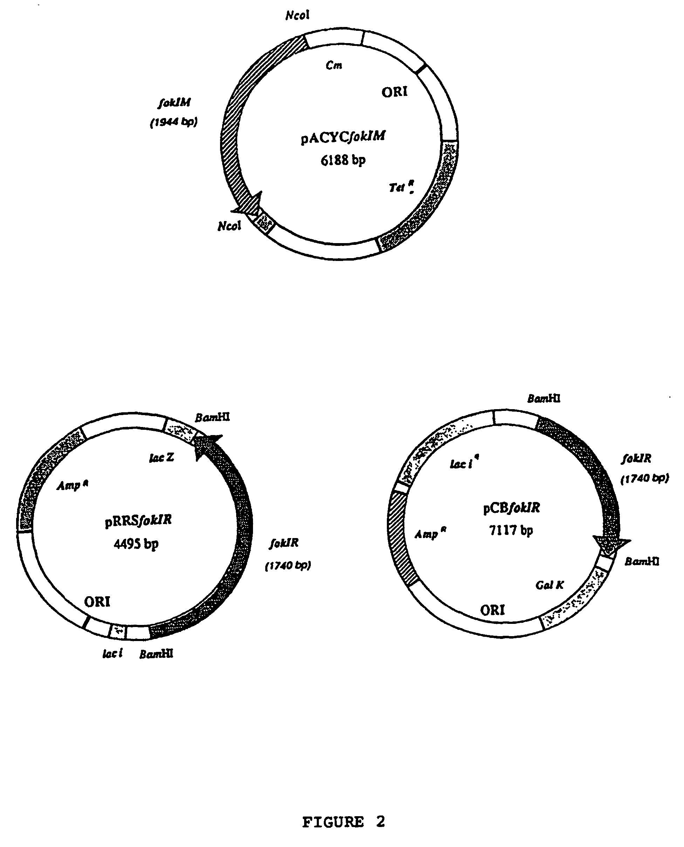

FIGURE 2 shows the structure of plasmids pACYC*MfokIM*, pRRS*RfokIR* and pCB*fokIR*. The PCR-modified *fokIM* gene was inserted at the *NcoI* site of pACYC184 to form pACYC*fokIM*. The PCR-generated *fokIR* gene was inserted at the *BamHI* sites of pRRS and pCB to form pRRS*fokIR* and pCB*fokIR*, respectively. pRRS possesses a *lac* UV5 promoter and pCB contains a strong *tac* promoter. In addition, these vectors contain the positive retroregulator sequence downstream of the inserted *fokIR* gene.

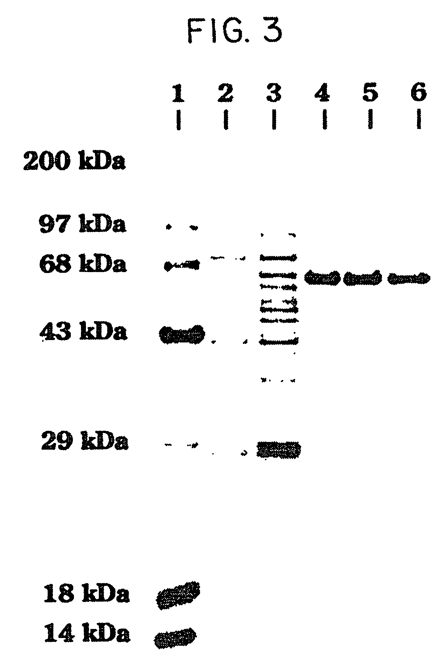

FIGURE 3 shows SDS (0.1%) - polyacrylamide (12%) gel electrophoretic profiles at each step in the purification of *Fok*I endonuclease. Lanes: 1, protein standards; 2, crude extract from uninduced cells; 3, crude extract from cells induced with 1 mM IPTG; 4, phosphocellulose pool; 5, 50-70% (NH₄)₂SO₄ fractionation pool; and 6, DEAE pool.

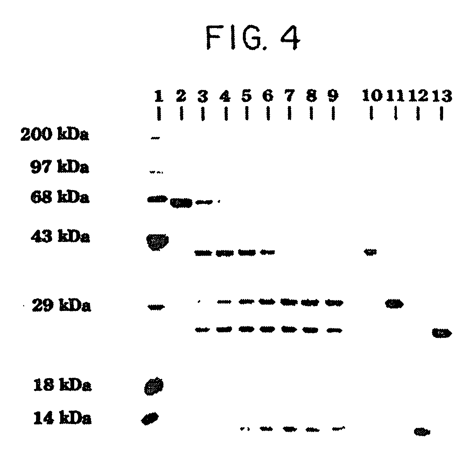

FIGURE 4 shows SDS (0.1%) - polyacrylamide (12%) gel electrophoretic profiles of tryptic fragments at various time points of trypsin digestion of *Fok*I endonuclease in presence of the oligonucleotide DNA substrate, d-5'-CCTCTGGATGCTCTC-3'(SEQ ID NO: 10): 5'-GAGAGCATCCAGAGG-3'(SEQ ID NO:11). Lanes: 1, protein standards; 2, *Fok*I endonuclease; 3, 2.5 min; 4, 5 min; 5, 10 min; 6, 20 min; 7, 40 min; 8, 80 min; 9, 160 min of trypsin digestion respectively. Lanes 10-13: HPLC purified tryptic fragments. Lanes: 10, 41 kDa fragment; 11, 30 kDa fragment; 12, 11 kDa fragment; and 13, 25 kDa fragment.

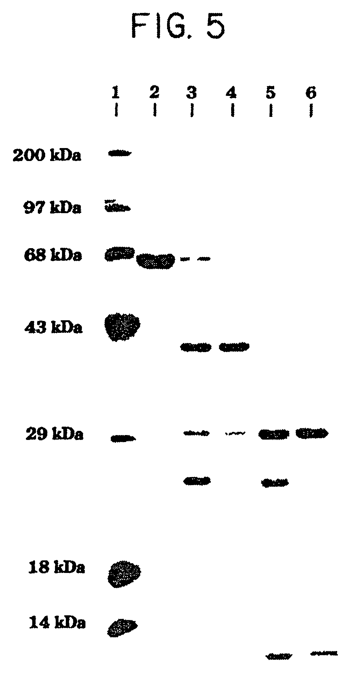

FIGURE 5 shows the identification of DNA binding tryptic fragments of *Fok*I endonuclease using an oligo dT-cellulose column. Lanes: 1, protein standards, 2, *Fok*I endonuclease; 3, 10 min trypsin digestion mixture of *Fok*I - oligo complex; 4, tryptic fragments that bound to the oligo dT-cellulose column; 5, 160 min trypsin digestion mixture of *Fok*I - oligo complex; 6, tryptic fragments that bound to the oligo dT-cellulose column.

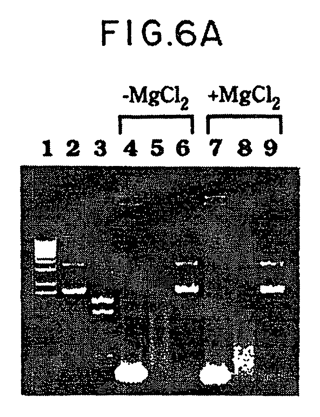

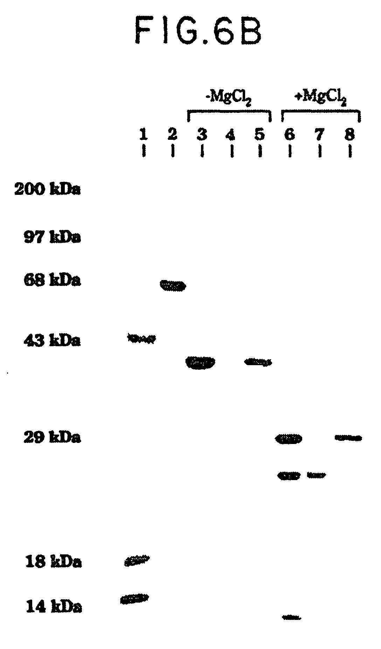

FIGURE 6 shows an analysis of the cleavage properties of the tryptic fragments of *Fok*I endonuclease.
   (A) The cleavage properties of the tryptic fragments were analyzed by agarose gel electrophoresis. 1 µg of pTZ19R in 10mM Tris.HCl (pH 8), 50mM NaCl, 1mM DTT, and 10mM MgCl₂ was digested with 2 µl of the solution containing the fragments (tryptic digests, breakthrough and eluate respectively) at 37°C for 1 hr in a reaction volume of 10 µl. Lanes 4 to 6 correspond to trypsin digestion of *FokI* - oligo complex in absence of MgCl₂. Lanes 7 to 9 correspond to trypsin digestion of *Fok*I - oligo complex in presence of 10 mM MgCl₂. Lanes: 1, 1 kb ladder; 2, pTZ19R; 3, pTZ19R digested with *Fok*I endonuclease; 4 and 6, reaction mixture of the tryptic digests of *Fok*I - oligo complex; 5 and 7, 25 kDa C-terminal fragment in the breakthrough volume; 6 and 9, tryptic fragments of *Fok*I that bound to the DEAE column. The intense bands at bottom of the gel correspond to excess oligonucleotides.
   (B) SDS (0.1%) - polyacrylamide (12%) gel electrophoretic profiles of fragments from the DEAE column. Lanes 3 to 5 correspond to trypsin digestion of *Fok*I - oligo complex in absence of MgCl₂. Lanes 6 to 8 correspond to trypsin digestion of *Fok*I - oligo complex in presence of 10 mM MgCl₂. Lanes: 1, protein standards; 2, *Fok*I endonuclease; 3 and 6, reaction mixture of the tryptic digests of *Fok*I - oligo complex; 4 and 7, 25 kDa C-terminal fragment in the breakthrough volume; 5 and 8, tryptic fragments of *Fok*I that bound to the DEAE column.

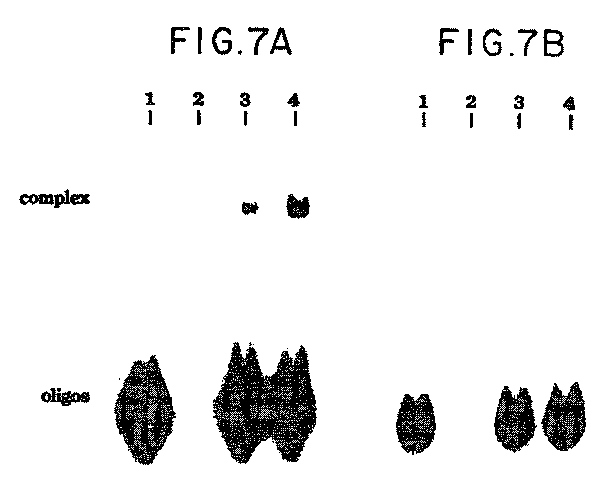

FIGURE 7 shows an analysis of sequence - specific binding of DNA by 41 kDa N-terminal fragment using gel mobility shift assays. For the exchange reaction, the complex (10 µl) was incubated with 1 µl of ³²P-labeled specific (or non-specific) oligonucleotide duplex in a volume of 20 µl containing 10 mM Tris.HCl, 50 mM NaCl and 10 mH MgCl₂ at 37°C for various times. 1 µl of the 5'-³²P-labeled specific probe [d-5'-CCTCTGGATGCTCTC-3'(SEQ ID NO: 10): 5'-GAGAGCATCCAGAGG-3' (SEQ ID NO: 11)] contained 12 picomoles of the duplex and ∼ 50 x 10³ cpm. 1µl of the 5'-³²P-labeled non-specific probe [5'-TAATTGATTCTTAA-3'(SEQ ID NO: 12):5'-ATTAAGAATCAATT-3' (SEQ ID NO: 13)] contained 12 picomoles of the duplex and - 25 x 10³ cpm. (A) Lanes: 1, specific oligonucleotide duplex; 2, 41 kDa N-terminal fragment-oligo complex; 3 and 4, specific probe incubated with the complex for 30 and 120 min respectively. (B) Lanes: 1, non-specific oligonucleotide duplex; 2, 41 kDa N-terminal fragment-oligo complex; 3 and 4 non-specific probe incubated with the complex for 30 and 120 min respectively.

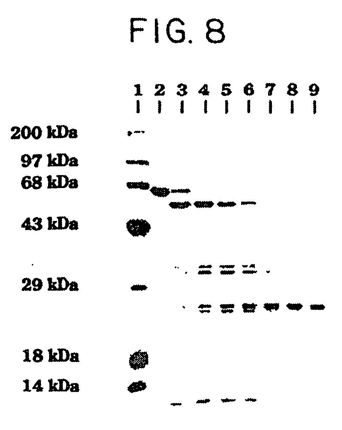

FIGURE 8 shows SDS (0.1%) polyacrylamide (12%) gel electrophoretic profiles of tryptic fragments at various time points of trypsin digestion of *Fok*I endonuclease. The enzyme (200 µg) in a final volume of 200 µl containing 10 mH Tris.HCl, 50 mM NaCl and 10mM MgCl₂ was digested with trypsin at RT. The trypsin to *Fok*I ratio was 1:50 by weight. Aliquots (28 µl) from the reaction mixture removed at different time intervals and quenched with excess antipain. Lanes: 1, protein standards; 2, *Fok*I endonuclease; 3, 2.5 min; 4, 5.0 min; 5, 10 min; 6, 20 min; 7, 40 min; 8, 80 min; and 9,160 min of trypsin digestion respectively.

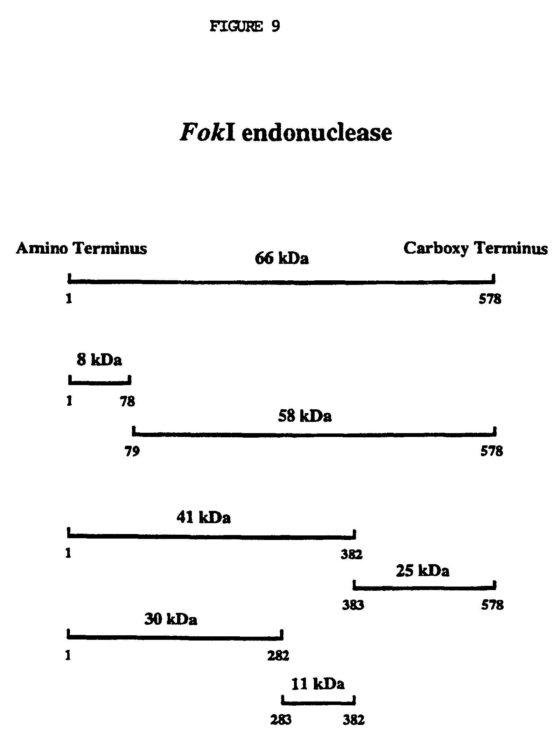

FIGURE 9 shows the tryptic map of *Fok*I endonuclease (A) *Fok*I endonuclease fragmentation pattern in absence of the oligonucleotide substrate. (B) *Fok*I endonuclease fragmentation pattern in presence of the oligonucleotide substrate.

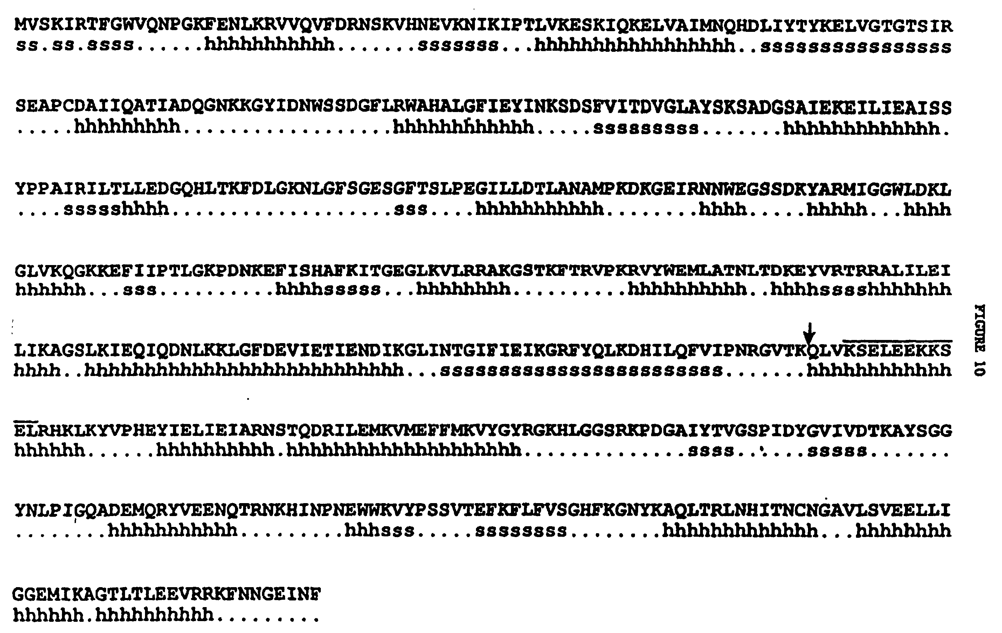

FIGURE 10 shows the predicted secondary structure of *Fok*I based on its primary sequencing using the PREDICT program. (See SEQ ID NO:31) The trypsin cleavage site of *FokI* in the presence of DNA substrates is indicated by the arrow. The KSELEEKKSEL segment is highlighted. The symbols are as follows: h, helix; s, sheet; and _{•}, random coil.

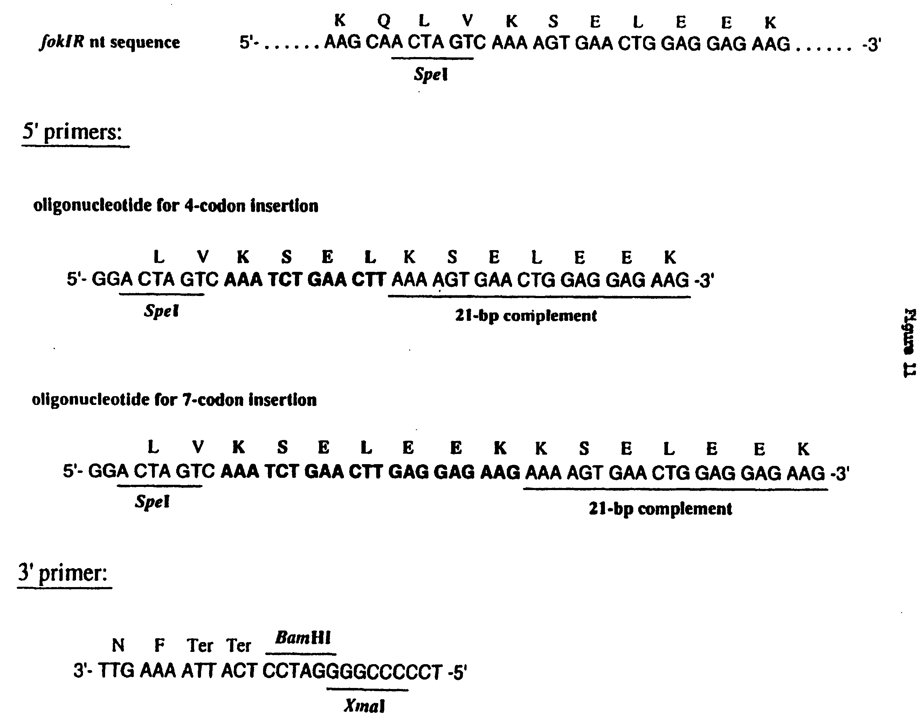

FIGURE 11 shows the sequences of the 5' and 3' oligonucleotide primers used to construct the insertion mutants of *Fok*I (see SEQ ID NO:32, SEQ ID NO:33, SEQ ID NO:34 SEQ ID NO:35, SEQ ID NO:36, SEQ ID NO:37, SEQ ID NO:38 and SEQ ID NO:39, respectively). The four and seven codon inserts are shown in bold letters. The amino acid sequence is indicated over the nucleotide sequence. The same 3' primer was used in the PCR amplification of both insertion mutants.

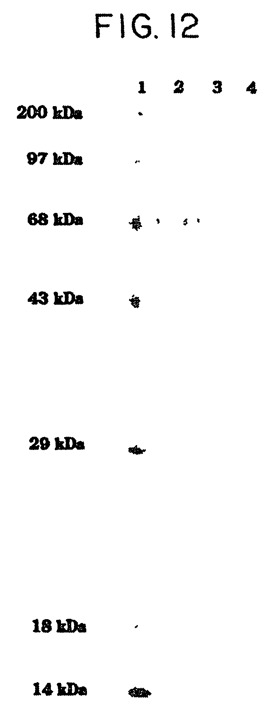

FIGURE 12 shows the SDS/PAGE profiles of the mutant enzymes purified to homogeneity. Lanes: 1, protein standards; 2, *Fok*I; 3, mutant *Fok*I with 4-codon insertion; and 4, mutant *FokI* with 7-codon insertion.

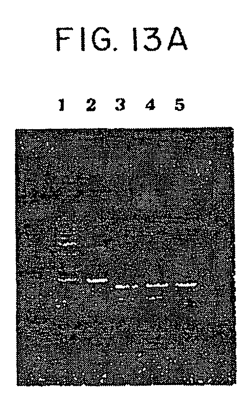

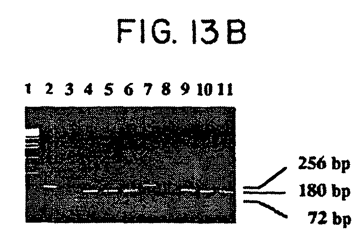

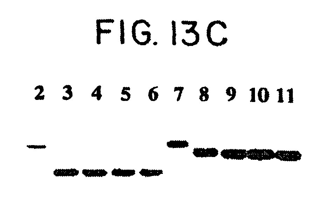

FIGURE 13 shows an analysis of the DNA sequence specificity of the mutant enzymes. The DNA substrates were digested in 10 mM Tris HCl, pH 8.0/50 mM MaCl/1 mM DTT/10mM MgCl₂ at 37°C for 2 hrs.
   (A) Cleavage pattern of pTZ19R DNA substrate analyzed by 1% agarose gel electrophoresis. 2µg of pTZ19R DNA was used in each reaction. Lanes: 1, 1-kilobase (kb) ladder; 2, pTZ19R; 3, pTZ19R digested with *Fok*I; pTZ19R digested with mutant *Fok*I with 4-codon insertion; and 5, pTZ19R digested with mutant *FokI* with 7-codon insertion.
   (B) Cleavage pattern of 256 bp DNA substrate containing a single *FokI* site analyzed by 1.5% agarose gel electrophoresis. 1µg of radiolabeled substrates (³²P-labeled on individual strands) was digested as described above. The agarose gel was stained with ethidium bromide and visualized under UV light. Lanes 2 to 6 correspond to the ³²P-labeled substrate in which the 5'-CATCC-3' strand is ³²-P labeled. Lanes 7 to 11 correspond to the substrate in which the 5'-GGATG-3' strand is ³²P-labeled. Lanes: 1, 1kb ladder; 2 and 7, ³²P-labeled 250 bp DNA substrates; 3 and 8, ³²-P labeled substrates cleaved with *Fok*I; 4 and 9, purified the laboratory wild-type *Fok*I; 5 and 10, mutant *Fok*I with 4-codon insertion; 6 and 11, mutant *Fok*I with 7-codon insertion.
   (C) Autoradiograph of the agarose gel from above. Lanes: 2 to 11, same as in B.

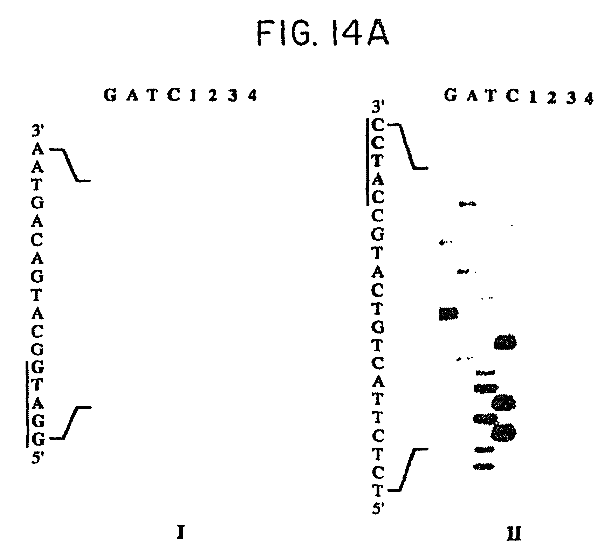

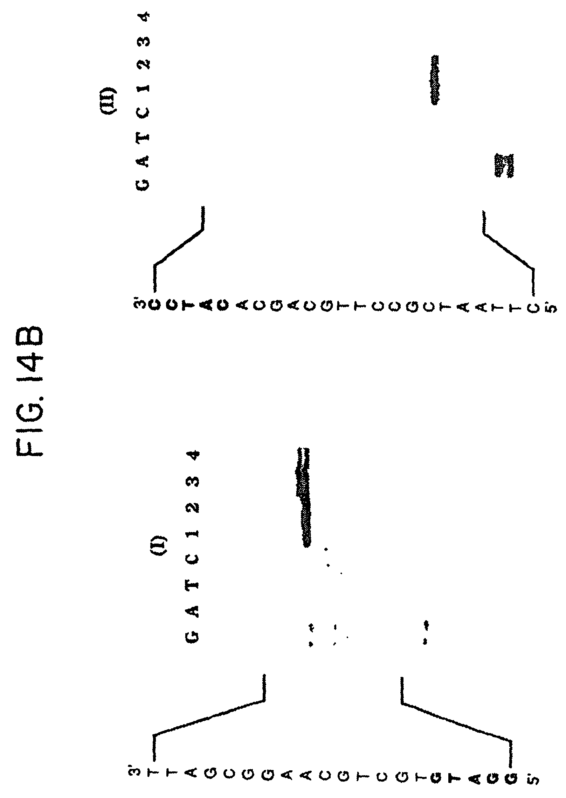

FIGURE 14 shows an analysis of the distance of cleavage from the recognition site by *FokI* and the mutant enzymes. The unphosphorylated oligonucleotides were used for dideoxy DNA sequencing with pTZ19R as the template. The sequencing products (G, A, T, C) were electrophoresed on a 6% acrylamide gel containing 7M urea, and the gel dried. The products were then exposed to an x-ray film for 2 hrs. Cleavage products from the 100 bp and the 256 bp DNA substrates are shown in A and B, respectively. I corresponds to substrates containing ³²P-label on the 5'-GGATG-3' strand, and II corresponds to substrates containing ³²P-label on the 5'-CATCC-3* strand. Lanes: 1, *Fok*I; 2, *Fok*I ; 3, mutant *Fok*I with 4-codon insertion; and 4, mutant *Fok*I with 7-codon insertion.

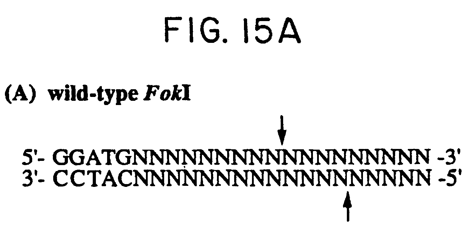

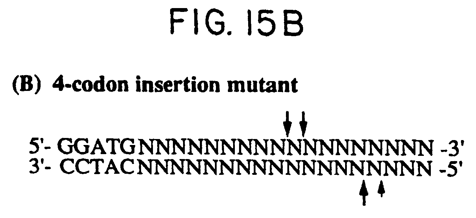

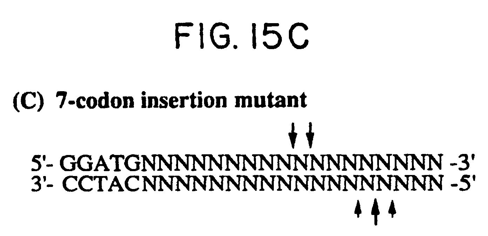

FIGURE 15 shows a map of the cleavage site(s) of *Fok*I and the mutant enzymes based on the 100 bp DNA substrate containing a single *Fok*I site: (A) wild-type *Fok*I; (B) mutant *Fok*I with 4-codon insertion; and (C) mutant *Fok*I with 7-codon insertion (see SEQ ID NO:40). The sites of cleavage are indicated by the arrows. Major cleavage sites are shown by larger arrows.

### DETAILED DESCRIPTION OF THE INVENTION

The present invention is based on the identification and characterization of the functional domains of the *Fok*I restriction endonuclease. In the experiments resulting in the present invention, it was discovered that the *Fok*I restriction endonuclease is a two domain system, one domain of which possesses the sequence-specific recognition activity while the other domain contains the nuclease cleavage activity.

The *Fok*I restriction endonuclease recognizes the non-palindromic pentanucleotide 5'-GGATG-3'(SEQ ID NO: 1):5'-CATCC-3'(SEQ ID NO: 2) in duplex DNA and cleaves 9/13 nucleotides downstream from the recognition site. Since 10 base pairs are required for one turn of the DNA helix, the present inventors hypothesized that the enzyme would interact with one face of the DNA by binding at one point and cleave at another point on the next turn of the helix. This suggested the presence of two separate protein domains, one for sequence-specific recognition of DNA and one for endonuclease activity. The hypothesized two domain structure was shown to be the correct structure of the *Fok*I endonuclease system by studies that resulted in the present invention.

Accordingly, the present invention relates to a DNA segment which encodes the N-terminus of the *Fok*I restriction endonuclease (preferably, about the N-terminal 2/3's of the protein). This DNA segment encodes a protein which has the sequence-specific recognition activity of the endonuclease, that is, the encoded protein recognizes the non-palindromic pentanucleotide d-5'-GGATG-3'(SEQ ID NO: 1):5'-CATCC-3'(SEQ ID NO: 2) in duplex DNA. Preferably, the DNA segment of the present invention encodes amino acids 1-382 of the *Fok*I endonuclease.

The present invention also relates to a DNA segment which encodes the C-terminus of the *Fok*I restriction endonuclease. The protein encoded by this DNA segment has the nuclease cleavage activity of the *Fok*I restriction endonuclease. Preferably, the DNA segment encodes amino acids 383-578 of the *Fok*I endonuclease. The DNA segments can be readily isolated from a biological samples using methods known in the art, for example, gel electrophoresis, affinity chromatography, polymerase chain reaction (PCR) or a combination thereof. Further, the DNA segments can be chemically synthesized using standard methods in the art.

The present invention also relates to the proteins encoded by the DNA segments. Thus, the present invention relates to a protein consisting essentially of the N-terminus of the *Fok*I endonuclease which retains the sequence-specific recognition activity of the enzyme. This protein has a molecular weight of about 41 kilodaltons as determined by SDS polyacrylamide gel electrophoresis in the presence of 2-mercaptoethanol.

The present invention relates to a protein consisting essentially of the C-terminus of the *Fok*I restriction endonuclease (preferably, the C-terminal 1/3 of the protein). The molecular weight of this protein is about 25 kilodaltons as determined by SDS polyacrylamide gel electrophoresis in the presence of 2-mercaptoethanol.

The proteins can be isolated or purified from a biological sample using methods known in the art. For example, the proteins can be obtained by isolating and cleaving the *Fok*I restriction endonuclease. Alternatively, the proteins can be chemically synthesized or produced using recombinant DNA technology and purified.

The DNA segments can be used to generate 'hybrid' restriction enzymes by linking other DNA binding protein domains with the nuclease domain of *Fok*I. This can be achieved chemically as well as by recombinant DNA technology. Such chimeric enzymes are useful for physical mapping and sequencing of genomes of various species, such as, humans, mice and plants. For example, such enzymes would be suitable for use in mapping the human genome.

Such chimeric enzymes are also valuable research tools in recombinant DNA technology and molecular biology. Currently only 4-6 base pair cutters and a few 8 base pair cutters are available commercially. (There are about 10 endonucleases which cut >6 base pairs that are available commercially.) By linking other DNA binding proteins to the nuclease domain of *Fok*I new enzymes can be generated that recognize more than 6 base pairs in DNA.

Accordingly, the present invention relates to a DNA construct and the hybrid restriction enzyme encoded therein. The DNA construct comprises a first DNA segment encoding the nuclease domain of the *Fok*I restriction endonuclease, a second DNA segment encoding a sequence-specific recognition domain and a vector. The first DNA segment and the second DNA segment are operably linked to the vector so that expression of the segments can be effected thereby yielding a chimeric restriction enzyme. The construct can comprise regulatory elements such as promoters (for example, T7, *tac, trp* and lac UV5 promoters), transcriptional terminators or retroregulators (for example, stem loops). Host cells (procaryotes such as *E. coli*) can be transformed with the DNA constructs and used for the production of chimeric restriction enzymes.

The hybrid enzymes of the present invention comprise the nuclease domain of *Fok*I linked to a recognition domain of another enzyme or DNA binding protein (such as, naturally occurring DNA binding proteins that recognize 6 base pairs). Suitable recognition domains include, but are not limited to, the recognition domains of zinc finger motifs; homeo domain motifs; other DNA binding protein domains of lambda repressor, *lac* repressor, *cro,* gal4; DNA binding protein domains of oncogenes such as *myc, jun*; and other naturally occurring sequence-specific DNA binding proteins that recognize >6 base pairs.

The hybrid restriction enzymes of the present invention can be produced by those skilled in the art using known methodology. For example, the enzymes can be chemically synthesized or produced using recombinant DNA technology well known in the art. The hybrid enzymes of the present invention can be produced by culturing host cells (such as, HB101, RR1, RB791 and MM294) containing the DNA construct of the present invention and isolating the protein. Further, the hybrid enzymes can be chemically synthesized, for example, by linking the nuclease domain of the *Fok*I to the recognition domain using common linkage methods known in the art, for example, using protein crosslinking agents such as EDC/NHS, DSP, etc.

While the *Fok*I restriction endonuclease was the enzyme studied in the following experiments, it is expected that other Type IIS endonucleases (such as, those listed in Table 2) will function using a similar two domain structure which one skilled in the art could readily determine based on the present invention.

Recently, *Sts*I, a heteroschizomer of *Fok*I has been isolated from *Streptococcus sanguis* (Kita et al., Nucleic Acids Research 20 (3)) 618, 1992). *Sts*I recognizes the same nonpalindromic pentadeoxyribonucleotide 5'-GGATG-3':5'-CATCC-3' as *Fok*I but cleaves 10/14 nucleotides downstream of the recognition site. The *Sts*I *RM* system has been cloned and sequenced (Kita et al., Nucleic Acids Research 20 (16) 4167-72, 1992). Considerable amino acid sequence homology (≃30%) has been detected between the endonucleases, *Fok*I and *Sts*I.

A particular embodiment of the invention relates to the construction of two insertion mutants of *Fok*I endonuclease using the polymerase chain reaction (PCR). In particular, this embodiment includes a DNA construct comprising a first DNA segment encoding the catalytic domain of a Type IIS endonuclease which contains the cleavage activity of the Type IIS endonuclease, a second DNA segment encoding a sequence-specific recognition domain other than the recognition domain of the Type IIS endonuclease, and a third DNA segment comprising one or more codons. The third DNA segment is inserted between the first DNA segment and the second DNA segment. The construct also includes a vector. The Type IIS endonuclease is *Fok*I restriction endonuclease.

Suitable recognition domains include, but are not limited to, zinc finger motifs, homeo domain motifs, DNA binding domains of repressors, DNA binding domains of oncogenes and naturally occurring sequence-specific DNA binding proteins that recognize >6 base pairs.

As noted above, the recognition domain of *Fok*I restriction endonuclease is at the amino terminus of *Fok*I endonuclease, whereas the cleavage domain is probably at the carboxyl terminal third of the molecule. It is likely that the domains are connected by a linker region, which defines the spacing between the recognition and the cleavage sites of the DNA substrate. This linker region of *Fok*I is susceptible to cleavage by trypsin in the presence of a DNA substrate yielding a 41-kDa amino-terminal fragment (The DNA binding domain) and a 25-kDa carboxyl-terminal fragment (the cleavage domain). Secondary structure prediction of *Fok*I endonuclease based on its primary amino acid sequence supports this hypothesis (see Figure 10). The predicted structure reveals a long stretch of alpha helix region at the junction of the recognition and cleavage domains. This helix probably constitutes the linker which connects the two domains of the enzyme. Thus, it was thought that the cleavage distance of *Fok*I from the recognition site could be altered by changing the length of this spacer (the alpha helix). Since 3.6 amino acids are required to form one turn of the alpha helix, insertion of either four codons or seven codons in this region would extend the pre-existing helix in the native enzyme by one or two turns, respectively. Close examination of the amino acid sequence of this helix region revealed the presence of two KSEL repeats separated by amino acids EEK (Figure 10) (see SEQ ID NO:21). The segments KSEL (4 codons) (see SEQ ID NO:22) and KSELEEK (7 codons) (see SEQ ID NO:23) appeared to be good choices for insertion within this helix in order to extend it by one and two turns, respectively. (See Examples X and XI.) Thus, genetic engineering was utilized in order to create mutant enzymes.

In particular, the mutants are obtained by inserting one or more, and preferably four or seven, codons between the recognition and cleavage domains of *Fok*I. More specifically, the four or seven codons are inserted at nucleotide 1152 of the gene encoding the endonuclease. The mutants have the same DNA sequence specificity as the wild-type enzyme. However, they cleave one nucleotide further away from the recognition site on both strands of the DNA substrates as compared to the wild-type enzyme.

Analysis of the cut sites of *Fok*I and the mutants, based on the cleavage of the 100 bp fragment, is summarized in Figure 15. Insertion of four (or seven) codons between the recognition and cleavage domains of *Fok*I is accompanied by an increase in the distance of cleavage from the recognition site. This information further supports the presence of two separate protein domains within the *Fok*I endonuclease: one for the sequence specific recognition and the other for the endonuclease activity. The two domains are connected by a linker region which defines the spacing between the recognition and the cleavage sites of the DNA substrate. The modular structure of the enzyme suggests it may be feasible to construct chimeric endonucleases of different sequence specificity by linking other DNA-binding proteins to the cleavage domain of the *Fok*I endonuclease.

In view of the above-information, another embodiment of the invention includes a procaryotic cell comprising a first DNA segment encoding the catalytic domain of a Type IIS endonuclease which contains the cleavage activity of the Type IIS endonuclease, a second DNA segment encoding a sequence-specific recognition domain other than the recognition domain of the Type IIS endonuclease, and a third DNA segment comprising one or more codons. The third DNA segment is inserted between the first DNA segment and the second DNA segment. The cell also includes a vector. Additionally, it should be noted that the first DNA segment and the second DNA segment are operably linked to the vector so that a single protein is produced. The third segment may consist essentially of four or seven codons.

The present invention also includes the protein produced by the procaryotic cell referred to directly above. In particular, the isolated protein consists essentially of the recognition domain of the FokI restriction endonuclease, the catalytic domain of the FokI restriction endonuclease, and amino acids encoded by the codons present in the third DNA segment.

The following non-limiting Examples are provided to describe the present invention in greater detail.

### EXAMPLES

The following materials and methods were utilized in the isolation and characterization of the *Fok*I restriction endonuclease functional domains as exemplified hereinbelow.

### Bacterial strains and plasmids

Recombinant plasmids were transformed into *E.coli* RB791 *i*^{*q*} cells which carry the *lac i*^{*q*} allele on the chromosome (Brent and Ptashne, PNAS USA, 78:4204-4208, 1981) or *E.coli* RR1 cells. Plasmid pACYC*fokIM* is a derivative of pACYC184 carrying the PCR-generated *fokIM* gene inserted into *NcoI* site. The plasmid expresses the *Fok*I methylase constitutively and was present in RB791 cells (or RR1 cells) whenever the *fokIR* gene was introduced on a separate compatible plasmid. The *FokI* methylase modifies *Fok*I sites and provides protection against chromosomal cleavage. The construction of vectors pRRS and pCB are described elsewhere (Skoglund et al., Gene, 88:1-5, 1990).

### Enzymes, biochemicals and oligos

Oligo primers for PCR were synthesized with an Applied Biosystem DNA synthesizer using cyanoethyl phosphoramidite chemistry and purified by reversed phase HPLC. Restriction enzymes were purchased from New England Biolabs. The DNA ligase IPTG were from Boehringer-Mannheim. PCR reagents were purchased as a Gene Amp Kit from Perkin-Elmer. Plasmid purification kit was from QIAGEN.

### Restriction enzyme assays

Cells from a 5-ml sample of culture medium were harvested by centrifugation, resuspended in 0.5 ml sonication buffer [50 mM Tris.HCl (pH 8), 14mM 2-mercaptoethanol], and disrupted by sonication (3 x 5 seconds each) on ice. The cellular debris was centrifuged and the crude extract used in the enzyme assay. Reaction mixtures (10 µl) contained 10mM Tris.HCl (pH 8), 10 mM MgCl₂, 7 mM 2-mercaptoethanol, 50 µg of BSA, 1 µg of plasmid pTZ19R (U.S. biochemicals) and 1µl of crude enzyme. Incubation was at 37°C for 15 min. tRNA (10 µg) was added to the reaction mixtures when necessary to inhibit non-specific nucleases. After digestion,
1 µl of dye solution (100 mM EDTA, 0.1% bromophenol blue, 0.1% xylene cyanol, 50% glycerol) was added, and the samples were electrophoresed on a 1% agarose gel. Bands were stained with 0.5 µg ethidium bromide/ml and visualized with 310-nm ultraviolet light.

### SDS/PAGE

Proteins were prepared in sample buffer and electrophoresed in SDS (0.1%)- polyacrylamide (12%) gels as described by Laemmli (Laemmli, Nature, 222:680-685, 1970). Proteins were stained with coomassie blue.

### Example I

### Cloning of FokI RM system

The *Fok*I system was cloned by selecting for the modification phenotype. *Flavobacterium okeanokoites* strain DNA was isolated by the method described by caserta et al. (Caserta et al., J. Biol. Chem., 262:4770-4777, 1987). Several *Flavobacterium okeanokoites* genome libraries were constructed in plasmids pBR322 and pUC13 using the cloning enzymes *PstI, Bam*HI *and Bgl*II. Plasmid library DNA (10 µg) was digested with 100 units of *Fok*I endonuclease to select for plasmids expressing *fokI*M+ phenotype.

Surviving plasmids were transformed into RR1 cells and transformants were selected on plates containing appropriate antibiotic. After two rounds of biochemical enrichment, several plasmids expressing the *fokIM*+ phenotype from these libraries were identified. Plasmids from these clones were totally resistant to digestion by *Fok*I.

Among eight transformants that were analyzed from the *F*. *okeanokoites* pBR322 *PstI* library, two appeared to carry the *fokIM* gene and plasmids from these contained a 5.5 kb *PstI* fragment. Among eight transformants that were picked from *F. okeanokoites* pBR322 *Bam*HI library, two appeared to carry the *fokIM* gene and their plasmids contained ∼ 18 kb *Bam*HI fragment. Among eight transformants that were analyzed from the *F. okeanokoites* genome *Bgl*II library in pUC13, six appeared to carry the *fokIM* gene. Three of these clones had a 8 kb *Bgl*II insert while the rest contained a 16 kb *Bgl*II fragment.

Plating efficiency of phage λ on these clones suggested that they also carried the *fokIR* gene. The clones with the 8-kb *Bgl*II insert appeared to be most resistant to phage infection. Furthermore, the *Fok*I endonuclease activity was detected in the crude extract of this clone after partial purification on a phosphocellulose column. The plasmid, pUC*fokIRM* from this clone was chosen for further characterization.

The 5.5 kb *PstI* fragment was transferred to M13 phages and the nucleotide sequences of parts of this insert determined using Sanger's sequencing method (Sanger et al., PNAS USA, 74:5463-5467, 1977). The complete nucleotide sequence of the *Fok*I RM system has been published by other laboratories (Looney et al., Gene, 80:193-208, 1989; Kita et al., Nucleic Acid Res., 17:8741-8753, 1989; Kita et al., J. Biol. Chem. 264:5751-5756, 1989).

### Example II

### Construction of an efficient overproducer clone of FokI endonuclease using polymerase chain reaction.

The PCR technique was used to alter transcriptional and translational signals surrounding the *fokIR* gene so as to achieve overexpression in *E.coli* (Skoglund et al., Gene, 88:1-5, 1990). The ribosome-binding site preceding the *fokIR* and *fokIM* genes were altered to match the consensus *E. coli* signal.

In the PCR reaction, plasmid pUC*fokIRM* DNA linearized with *Bam*HI was used as the template. PCR reactions (100 µl) contained 0.25 nmol of each primer, 50 µM of each dNTP, 10 mM Tris.HCl (pH 8.3 at 25°C), 50 mM KCl, 1.5 mM MgCl₂ 0.01% (W/V) gelatin, 1 ng of template DNA, 5 units of Tag DNA polymerase. The oligo primes used for the amplification of the *fokIR* and *fokIM* genes are shown in Figure 1. Reaction mixtures (ran in quadruplicate) were overlayed with mineral oil and reactions were carried out using Perkin-Elmer-Cetus Thermal Cycler.

Initial template denaturation was programmed for 2 min. Thereafter, the cycle profile was programmed as follows: 2 min at 37°C (annealing), 5 min at 72°C (extension), and 1 min at 94°C (denaturation). This profile was repeated for 25 cycles and the final 72°C extension was increased to 10 min. The aqueous layers of the reaction mixtures were pooled and extracted once with 1:1 phenol/chloroform and twice with chloroform. The DNA was ethanol-precipitated and resuspended in 20 µl TE buffer [10 mM Tris.HCl, (pH 7.5), 1 mM EDTA]. The DNA was then cleaved with appropriate restriction enzymes to generate cohesive ends and gel-purified.

The construction of an over-producer clone was done in two steps. First, the PCR-generated DNA containing the *fokIM* gene was digested with *NcoI* and gel purified. It was then ligated into *NcoI*-cleaved and dephosphorylated pACYC184 and the recombinant DNA transfected into *E.coli* RB791 *i*^{*q*} or RR1 cells made competent as described by Maniatis et al (Maniatis et al., Molecular Cloning. A laboratory manual Cold Spring Harbor Laboratory, Cold Spring Harbor, NY, 1982). After Tc selection, several clones were picked and plasmid DNA was examined by restriction analysis for the presence of *fokIM* gene fragment in correct orientation to the chloramphenicol promoter of the vector (see figure 2). This plasmid expresses *Fok*I methylase constitutively and then protects the host from chromosomal cleavage, when the *fokIR* gene is introduced into this host on a compatible plasmid. The plasmid DNA from these clones are therefore resistant to *Fok*I digestion.

Second, the PCR-generated *fokIR* fragment was ligated into *Bam*HI-cleaved and dephosphorylated high expression vectors pRRS or pCB. pRRS possesses a *lac* UV5 promoter and pCB containing the strong tac promoter. In addition, these vectors contain the positive retroregulator stem-loop sequence derived from the crystal protein-encoding gene of *Bacillus Thuringiensis* downstream of the inserted *fokIR* gene. The recombinant DNA was transfected into competent *E.coli* RB791 *i*^{*q*} [pACYC*fokIM*] or RR1[pACYC*fokIM*]cells. After Tc and Ap antibiotic selection, several clones were picked and plasmid DNA was examined by restriction analysis for *fokIR* gene fragment in correct orientation for expression from the vector promoters. These constructs were then examined for enzyme production.

To produce the enzyme, plasmid-containing RB791 *i*^{*q*} or RR1 cells were grown at 37°C with shaking in 2x concentrated TY medium [1.6% tryptone, 1% yeast extract, 0.5% NaCl (pH 7.2)] supplemented with 20 µg Tc/ml (except for the pUC*fokIRM* plasmid) and 50 µg Ap/ml. IPTG was added to a concentration of 1 mM when the cell density reached O.D.₆₀₀ = 0.8. The cells were incubated overnight (12 hr) with shaking. As is shown in Figure 2, both constructs yield *Fok*I to a level of 5-8% of the total cellular protein.

### Examples III

### Purification of FoKI endonuclease

A simple three-step purification procedure was used to obtain electrophoretically homogeneous *Fok*I endonuclease. RR1 [pACYC*fokIM*, pRRS*fokIR*] were grown in 6L of 2 x TY containing 20µg Tc/ml and 50 µg/Ap ml at 37°C to A₆₀₀ = 0.8. and then induced overnight with 1 mM IPTG. The cells were harvested by centrifugation and then resuspended in 250 ml of buffer A [10 mM Tris.phosphate (pH 8.0), 7 mM 2-mercaptoethanol, 1 mM EDTA, 10% glycerol] containing 50 mM NaCl.

The cells were disrupted at maximum intensity on a Branson Sonicator for 1 hr at 4°C. The sonicated cells were centrifuged at 12,000 g for 2 hr at 4°C. The supernatant was then diluted to 1L with buffer A containing 50 mM NaCl. The supernatant was loaded onto a 10 ml phosphocellulose (Whatman) column pre-equilibrated with buffer A containing 50 mM NaCl. The column was washed with 50 ml of loading buffer and the protein was eluted with a 80-ml total gradient of 0.05M to 0.5M NaCl in buffer A. The fractions were monitored by A₂₈₀ absorption and analyzed by electrophoresis on SDS (0.1%)-polyacrylamide (12%) gels (Laemmli, Nature, 222:680-685, 1970). Proteins were stained with coomassie blue.

Restriction endonuclease activity of the fractions were assayed using pTZ19R as substrate. The fractions containing *Fok*I were pooled and fractionated with ammonium sulfate. The 50-70% ammonium sulfate fraction contained the *Fok*I endonuclease. The precipitate was resuspended in 50 ml of buffer A containing 25 mM NaCl and loaded onto a DEAE column. *Fok*I does not bind to DEAE while many contaminating proteins do. The flow-through was concentrated on a phosphocellulose column. Further purification was achieved using gel filtration (AcA 44) column. The *Fok*I was purified to electrophoretic homogeneity using this procedure.

SDS (0.1%) polyacrylamide (12%) gel electrophoresis profiles of protein species present at each stage of purification are shown in Figure 3. The sequence of the first ten amino acids of the purified enzyme was determined by protein sequencing. The determined sequence was the same as that predicted from the nucleotide sequence. Crystals of this purified enzyme have also been grown using PEG 4000 as the precipitant. *Fok*I endonuclease was purified further using AcA44 gel filtration column.

### Example IV

### Analysis of FokIR endonuclease by trypsin cleavage in the presence of DNA substrate.

Trypsin is a serine protease and it cleaves at the C-terminal side of lysine and arginine residues. This is a very useful enzyme to study the domain structure of proteins and enzymes. Trypsin digestion of *Fok*I in the presence of its substrate, d-5'-CCTCTGGATGCTCTC-3'(SEQ ID NO: 10): 5'-GAGAGCATCCAGAGG-3' (SEQ ID NO: 11) was carried out with an oligonucleotide duplex to *Fok*I molar ratio of 2.5:1. *Fok*I (200 µg) was incubated with the oligonucleotide duplex in a volume 180 µl containing 10 mM Tris.HCl, 50 mM NaCl, 10% glycerol and 10 mM MgCl₂ at RT for 1 hr. Trypsin (20 µl, 0.2 mg/ml) was added to the mixture. Aliquots (28 µl) from the reaction mixture were removed at different time intervals and quenched with excess trypsin inhibitor, antipain. The tryptic fragments were purified by reversed-phase HPLC and their N-terminus sequence determined using an automatic protein sequenator from Applied Biosystems.

The time course of trypsin digestion of *Fok*I endonuclease in the presence of 2.5 molar excess of oligonucleotide substrate and 10 mH MgCl₂ is shown in Figure 4. At the 2.5 min time point only two major fragments other than the intact *Fok*I were present, a 41 kDa fragment and a 25 kDa fragment. Upon further trypsin digestion, the 41 kDa fragment degraded into a 30 kDa fragment and 11 kDA fragment. The 25 kDa fragment appeared to be resistant to any further trypsin digestion. This fragment appeared to be less stable if the trypsin digestion of *Fok*I - oligo complex was carried out in the absence of MgCl₂.

Only three major fragments (30 kDa, 25 kDa and 11 kDa) were present at the 160 min time point. Each of these fragments (41 kDa, 30 kDa, 25 kDa and 11 kDa) was purified by reversed-phase HPLC and their N-terminal amino acid sequence were determined (Table I). By comparing these N-terminal sequences to the predicted sequence of *Fok*I, the 41 kDa and 25 kDa fragments were identified as N-terminal and C-terminal fragments, respectively. In addition, the 30 kDa fragment was N-terminal.

### Example V

### Isolation of DNA binding tryptic fragments of FokI endonuclease using oligo dT-cellulose affinity column.

The DNA binding properties of the tryptic fragments were analyzed using an oligo dT-cellulose column. *Fok*I (160 µg) was incubated with the 2.5 molar excess oligonucleotide duplex [d-5'-CCTCTGGATGCTCTC(A)₁₅-3' (SEQ ID NO: 14): 5'GAGAGCATCCAGAGG(A)₁₅-3' (SEQ ID NO: 15)] in a volume of 90 µl containing 10 mM Tris.HCl (pH 8), 50 mM NaCl, 10% glycerol and 10 mM MgCl₂ at RT for 1 hr. Trypsin (10 µl, 0.2 mg/ml) was added to the solution to initiate digestion. The ratio of trypsin to *Fok*I (by weight) was 1:80. Digestion was carried out for 10 min to obtain predominantly 41 kDa N-terminal fragment and 25 kDa C-terminal fragments in the reaction mixture. The reaction was quenched with large excess of antipain (10 µg) and diluted in loading buffer [10 mM.Tris HCl (pH 8.0), 1 mM EDTA and 100 mM MgCl₂] to a final volume of 400 µl.

The solution was loaded onto a oligo dT-cellulose column (0.5 ml, Sigma, catalog #0-7751) pre-equilibrated with the loading buffer. The breakthrough was passed over the oligo dT-cellulose column six times. The column was washed with 5 ml of loading buffer and then eluted twice with 0.4 ml of 10 mM Tris.HCl (pH 8.0), 1 mM EDTA. These fractions contained the tryptic fragments that were bound to the oligonucleotide DNA substrate. The tryptic fragment bound to the oligo dT-cellulose column was analyzed by SDS-polyacrylamide gel electrophoresis.

In a separate reaction, the trypsin digestion was carried out for 160 min to obtain predominantly the 30 kDa, 25 kDa and 11 kDa fragments in the reaction mixture.

Trypsin digestion of *Fok*I endonuclease for 10 min yielded the 41 kDa N-terminal fragment and 25 kDa c-terminal fragments as the predominant species in the reaction mixture (Figure 5, Lane 3). When this mixture was passed over the oligo dT-cellulose column, only the 41 kDa N-terminal fragment is retained by the column suggesting that the DNA binding property of *Fok*I endonuclease is in the N-terminal 2/3's of the enzyme. The 25 kDa fragment is not retained by the oligo dT-cellulose column.

Trypsin digestion of *Fok*I - oligo complex for 160 min yielded predominantly the 30 kDa, 25 kDa and 11 kDa fragments (Figure 5, Lane 5). When this reaction mixture was passed over oligo dT-cellulose column, only the 30 kDa and 11 kDa fragments were retained. It appears these species together bind DNA and they arise from further degradation of 41 kDa N-terminal fragment. The 25 kDa fragment was not retained by oligo dT-cellulose column. It also did not bind to DEAE and thus could be purified by passage through a DEAE column and recovering it in the breakthrough volume.

*Fok*I (390 µg) was incubated with 2.5 molar excess of oligonucleotide duplex [d-5'-CTCTGGATGCTCTC-3 (SEQ ID NO: 10)':5'-GAGAGCATCCAGAGG-3'(SEQ ID NO: 11)] in a total volume of 170 µl containing 10 mM Tris.HCl (pH 8), 50 mM NaCl and 10% glycerol at RT for 1 hr. Digestion with trypsin (30 µl; 0.2 mg/ml) in the absence of MgCl₂ was for 10 min at RT to maximize the yield of the 41 kDa N-terminal fragment. The reaction was quenched with excess antipain (200 µl). The tryptic digest was passed through a DEAE column. The 25 kDa of C-terminal fragment was recovered in the breakthrough volume. All the other tryptic fragments (41 kDa, 30 kDa and 11 kDa) were retained by the column and were eluted with 0.5M NaCl buffer (3 x 200 µl). In a separate experiment, the trypsin digestion of *Fok*I -oligo complex was done in presence of 10 mM MgCl₂ at RT for 60 min to maximize the yield of 30 kDa and 11 kDa fragments. This purified fragment cleaved non-specifically both unmethylated DNA substrate (pTZ19R; Figure 6) and methylated DNA substrate (pACYC*fokIM*) in the presence of MgCl₂. These products are small, indicating that it is relatively non-specific in cleavage. The products were dephosphorylated using calf intestinal phosphatase and rephosphorylated using polynucleotide kinase and [^{γ}-³²P] ATP. The ³²P-labeled products were digested to mononucleotides using DNase I and snake venom phosphodiesterase. Analysis of the mononucleotides by PEI-cellulose chromatography indicates that the 25 kDa fragment cleaved preferentially phosphodiester bonds 5' to G>A>>T∼C. The 25 kDa C-terminal fragment thus constitutes the cleavage domain of *Fok*I endonuclease.

The 41 kDa N-terminal fragment - oligo complex was purified by agarose gel electrophoresis. *Fok*I endonuclease (200 µg) was incubated with 2.5 molar excess of oligonucleotide duplex, [d-5'-CCTCTGGATGCTCTC-3'(SEQ ID NO: 10): 5'-GAGAGCATCCAGAGG-3'(SEQ ID NO:11)] in a volume of 180 µl containing 10 mM Tris.HCl (pH 8.0), 50 mM NaCl and 10% glycerol at RT for 1 hr. Tracer amounts of ³²P-labeled oligonucleotide duplex was incorporated into the complex to monitor it during gel electrophoresis. Digestion with trypsin (20 µl; 0.2 mg/ml) was for 12 min at RT to maximize the yield of the 41 kDa N-terminal fragment. The reaction was quenched with excess antipain. The 41 kDa N-terminal fragment - oligo complex was purified by agarose gel electrophoresis. The band corresponding to the complex was excised and recovered by electroelution in a dialysis bag (∼ 600 µl). Analysis of the complex by SDS-PAGE revealed 41 kDa N-terminal fragment to be the major component. The 30 kDa N-terminal fragment and the 11 kDa C-terminal fragment were present as minor components. These together appeared to bind DNA and co-migrate with the 41 kDa N-terminal fragment-oligo complex.

The binding specificity of the 41 KDa N-terminal fragment was determined using gel mobility shift assays.

### Example VI

### Gel Mobility shift assays

The specific oligos (d-5'-CCTCTGGATGCTCTC-3'(SEQ ID NO: 10) and d-5'-GAGAGCATCCAGAGG-3' (SEQ ID NO: 11)) were 5'-³²P-labeled in a reaction mixture of 25 µl containing 40 mM Tris.HCl(pH7.5), 20mM MgCl₂,50 mM NaC1, 10 mM DTT, 10 units of T4 polynucleotide kinase (from New England Biolabs) and 20 µCi[^{γ}-³²P] ATP (3000 Ci/mmol). The mixture was incubated at 37°C for 30 min. The kinase was inactivated by heating the reaction mixture to 70°C for 15 min. After addition of 200 µl of water, the solution was passed through Sephadex G-25 (Superfine) column (Pharmacia) to remove the unreacted [^{γ}-³²P] ATP. The final concentration of labeled single-strand oligos were 27 µM.

The single-strands were then annealed to form the duplex in 10 mM Tris.HCl (pH 8.0), 50 mM NaCl to a concentration of 12 µM. 1 µl of the solution contained - 12 picomoles of oligo duplex and ∼ 50 x 10³cpm. The non-specific oligos (d-5'-TAATTGATTCTTAA-3' (SEQ ID NO: 12) and d-5'-ATTAAGAATCAATT-3'(SEQ ID NO:13)) were labeled with [^{γ}-³²P]ATP and polynucleotide kinase as described herein. The single-stranded oligos were annealed to yield the duplex at a concentration of 12µM. 1 µl of the solution contained ∼ 12 picomoles of oligo duplex and ∼ 25 x 10³cpm. The non-specific oligos (d-5'-TAATTGATTCTTAA-3'(SEQ ID NO: 12) and d-5'-ATTAAGAATCAATT-3' (SEQ ID NO: 13)) were labeled with [^{γ}-³²P] ATP and polynucleotide Kinase as described herein. The single-strand oligos were annealed to yield the duplex at a concentration of 12µM. 1 µl of the solution contained 42 picomdes of oligo duplex and ∼25x10³ cpm.

10 µl of 41 kDa N-terminal fragment-oligo complex (∼ 2 pmoles) in 10 mM Tris.HCl, 50 mM NaCl and 10 mM MgCl₂ was incubated with 1 µl of ³²P-labeled specific oligonucleotide duplex (or ³²P-labeled non-specific oligonucleotide duplex) at 37°C for 30 min and 120 min respectively. 5 µl of 75% glycerol was added to each sample and loaded on a 8% nondenaturing polyacrylamide gel. Electrophoresis was at 300 volts in TBE buffer until bromophenol blue moved ∼ 6 cm from the top of the gel. The gel was dried and autoradiographed.

The complex readily exchanged ³²P-labeled specific oligonucleotide duplex that contained the *Fok*I recognition site as seen from the gel mobility shift assays (Figure 7). It did not, however, exchange the ³²P-labeled non-specific oligonucleotide duplex that did not contain the *Fok*I recognition site. These results indicate that all the information necessary for sequence-specific recognition of DNA are encoded within the 41 kDa N-terminal fragment of *Fok*I.

### Example VII

### Analysis of FokI by trypsin cleavage in the absence of DNA substrate.

A time course of trypsin digestion of *Fok*I endonuclease in the absence of the DNA substrate is shown in Figure 8. Initially, *Fok*I cleaved into a 58 kDa fragment and a 8 kDa fragment. The 58 kDa fragment did not bind DNA substrates and is not retained by the oligo dT-cellulose column. On further digestion, the 58 kDa fragment degraded into several intermediate tryptic fragments. However, the complete trypsin digestion yielded only 25 kDa fragments (appears as two overlapping bands).

Each of these species (58 kDa, 25 kDa and 8 kDa) were purified by reversed phase HPLC and their amino terminal amino acid sequence determined (Table I). Comparison of the N-terminal sequences to the predicted *Fok*I sequence revealed that the 8 kDa fragment to be N-terminal and the 58 kDa fragment to be C-terminal. This further supports the conclusion that N-terminus of *Fok*I is responsible for the recognition domain. Sequencing the N-terminus of the 25 kDa fragments revealed the presence of two different components. A time course of trypsin digestion of *Fok*I endonuclease in a the presence of a non-specific DNA substrate yielded a profile similar to the one obtained when trypsin digestion of *Fok*I is carried out in absence of any DNA substrate.

### Example VIII

### Cleavage specificity of the 25 kDa C-terminal tryptic fragment of FokI

The 25 kDa C-terminal tryptic fragment of *Fok*I cleaved pTZ19R to small products indicating non-specific cleavage. The degradation products were dephosphorylated by calf intestinal phosphatase and ³²P-labeled with the polynucleotide kinase and [τ-³²P]ATP. The excess label was removed using a Sephadex G-25 (Superfine) column. The labeled products were then digested with 1 unit of pancreatic DNase I (Boehringer-Mannheim) in buffer containing 50 mM Tris.HCl(pH7.6), 10mM MgCl₂ at 37°C for 1 hr. Then, 0.02 units of snake venom phosphodiesterase was added to the reaction mixture and digested at 37°C for 1 hr.

### Example IX

### Functional domains in FokI restriction endonuclease.

Analysis of functional domains of *Fok*I (in the presence and absence of substrates) using trypsin was summarized in Figure 9. Binding of DMA substrate by *Fok*I was accompanied by alteration in the structure of the enzyme. This study supports that presence of two separate protein domains within this enzyme: one for sequence-specific recognition and the other for endonuclease activity. The results indicate that the recognition domain is at the N-terminus of the FokI endonuclease, while the cleavage domain is probably in the C-terminus third of the molecule.

### Examples Relating to Construction of Insertion Mutants (X-XIV)

The complete nucleotide sequence of the *Fok*I *RM* system has been published by various laboratories (Looney et al., Gene 80: 193-208, 1989 & Kita et al., J. Biol.Chem. 264: 5751-56, 1989). Experimental protocols for PCR are described, for example, in Skoglund et al., Gene 88:1-5, 1990 and in Bassing et al., Gene 113:83-88, 1992. The procedures for cell growth and purification of the mutant enzymes are similar to the ones used for the wild-type *Fok*I (Li et al., Proc. Nat'l. Acad. Sci. USA 89:4275-79, 1992). Additional steps which include Sephadex G-75 gel filtration and Heparin-Sepharose CL-6B column chromatography were necessary to purify the mutant enzymes to homogeneity.

### Example X

### Mutagensis of SpeI Site at Nucleotide 162 within the fokIR Gene

The two step PCR technique used to mutagenize one of the *Spe*I sites within the *fokIR* gene is described in Landt et al., Gene 96: 125-28, 1990. The three synthetic primers for this protocol include: 1) the mutagenic primer (5'-TCATAA TAGCAACTAATTCTTTTTGGATCTT-3') (see SEQ ID NO:24) containing one base mismatch within the *Spe*I site; 2) the other primers each of which are flanked by restriction sites *Cla*I (5'-CCATCGATATAGCCTTTTTTATT-3') (see SEQ ID NO:25) and *XbaI* (5'-GCTCTAGAGGATCCGGAGGT-3') (see SEQ ID NO:26), respectively. An intermediate fragment was amplified using the *Xba*I primer and the mutagenic primer during the first step. The *Cla*I primer was then added to the intermediate for the second step PCR. The final 0.3 kb PCR product was digested with *Xba*I/*Cla*I to generate cohesive ends and gel-purified. The expression vector (pRRS*fokIR*) was cleaved with *Xba*I/*Cla*I. The large 4.2 kb fragment was then gel-purified and ligated to the PCR fragment. The recombinant DNA was transfected into competent *E. coli* RR1[pACYC*fokIM*] cells. After tetracycline and ampicillin antibiotic selection several clones were picked, and their plasmid DNA was examined by restriction analysis. The *Spe*I site mutation was confirmed by sequencing the plasmid DNA using Sanger's sequencing method (Sanger et al. Proc. Natl. Acad. Sci. USA 74: 5463-67, 1977).

### Example XI

### Construction of four (or seven) codon Insertion Mutants

The PCR-generated DNA containing a four (or seven) codon insertion was digested with a *Spe*I/*Xma*I and gel-purified. The plasmid, pRRS*fokIR* from Example X was cleaved with *Spe*I/*Xma*I, and the large 3.9 kb fragment was gel-purified and ligated to the PCR product. The recombinant DNA was transfected into competent RR1[pACYC*fokIM*] cells, and the desired clones identified as described in Example X. The plasmids from these clones were isolated and sequenced to confirm the presence of the four (or seven) codon insertion within the *fokIR* gene.

In particular, the construction of the mutants was performed as follows: (1) There are two *Spe*I sites at nucleotides 162 and 1152, respectively, within the *fokIR* gene sequence. The site at 1152 is located near the trypsin cleavage site of *Fok*I that separates the recognition and cleavage domains. In order to insert the four (or seven) codons around this region, the other *Spe*I site at 162 was mutagenized using a two step PCR technique (Landt et al. Gene 96:125-28, 1990). Introduction of this *Spe*I site mutation in the *fokIR* gene does not affect the expression levels of the overproducer clones. (2) The insertion of four (or seven) codons was achieved using the PCR technique. The mutagenic primers used in the PCR amplification are shown in Figure 11. Each primer has a 21 bp complementary sequence to the *fokIR* gene. The 5' end of these primers are flanked by *Spe*I sites. The codons for KSEL and KSELEEK repeats are incorporated between the *Spe*I site and the 21 bp complement. Degenerate codons were used in these repeats to circumvent potential problems during PCR amplification. The other primer is complementary to the 3' end of the *fokIR* gene and is flanked by a *XmaI* site. The PCR-generated 0.6 kb fragments containing the four (or seven) codon inserts digested with *SpeI*/*XmaI* and gel-purified. These fragments were substituted into the high expression vector pRRS*fokI*R to generate the mutants. Several clones of each mutant identified and their DNA sequence confirmed by Sanger's dideoxy chain termination method (Sanger et al. Proc. Natl. Acad. Sci. USA 74.5463-67 1977).

Upon induction with 1 mM isopropyl β-D-thiogalactoside (IPTG), the expression of mutant enzymes in these clones became most prominent at 3 hrs as determined by SDS/PAGE. This was further supported by the assays for the enzyme activity. The levels of expression of the mutant enzymes in these clones were much lower compared to the wild-type *Fok*I. IPTG induction for longer times resulted in lower enzyme levels indicating that the mutant enzymes were actively degraded within these clones. This suggests that the insertion of four (or seven) codons between the recognition and cleavage domains of *FokI* destabilizes the protein conformation making them more susceptible to degradation within the cells. SDS/PAGE profiles of the mutant enzymes are shown in Figure 12.

### Example XII

### Preparation of DNA Substrates with a Single FokI Site

Two substrates, each containing a single *Fok*I recognition site, were prepared by PCR using pTZ19R as the template. Oligonucleotide primers, 5'-CGCAGTGTTATCACTCAT-3' and 5'-CTTGGTTGAGTACTCACC-3'(see SEQ ID NO:27 and SEQ ID NO:28, respectively), were used to synthesize the 100 bp fragment. Primers, 5'-ACCGAGCTCGAATTCACT-3' and 5'-GATTTCGGCCTATTGGTT-3' (see SEQ ID NO:29 and SEQ ID NO:30 respectively), were used to prepare the 256 bp fragment. Individual strands within these substrates were radiolabled by using the corresponding ³²P-labeled phosphorylated primers during PCR. The products were purified from low-melting agarose gel, ethanol precipitated and resuspended in TE buffer.

### Example XIII

### Analysis of the Sequence Specificity of the Mutant Enzymes

The agarose gel electrophoretic profile of the cleavage products of pTZ19R DNA by FokI and the mutants are shown in Figure 13A. They are very similar suggesting that insertion of four (or seven) codons in the linker region between the recognition and cleavage domains does not alter its DNA sequence specificity. This was further confirmed by using ³²P-labeled DNA substrates (100 bp and 256 bp) each containing a single *Fok*I site. Substrates containing individual strands labeled with ³²P were prepared as described in Example XII. *Fok*I cleaves the 256 bp substrate into two fragments, 180 bp and 72 bp, respectively (Figure 13B). The length of the fragments was calculated from the ³²P-labeled 5' end of each strand. The autoradiograph of the agarose gel is shown in Figure 13C. Depending on which strand carries the ³²P-label in the substrate, either 72 bp fragment or 180 bp fragment appears as a band in the autoradiograph. The mutant enzymes reveal identical agarose gel profiles and autoradiograph. Therefore, insertion of four (or seven) codons between the recognition and cleavage domains does not alter the DNA recognition mechanism of *Fok*I endonuclease.

### Example XIV

### Analysis of the Cleavage Distances from the Recognition Site by the Mutant Enzymes

To determine the distance of cleavage by the mutant enzymes, their cleavage products of the ³²P-labeled substrates were analyzed by PAGE (Figure 14). The digests were analyzed alongside the sequencing reactions of pTZ19R performed with the same primers used in PCR to synthesize these substrates. The cleavage pattern of the 100 bp fragment by *Fok*I and the mutants are shown in Figure 14A. The cut sites are shifted from the recognition site on both strands of the substrates in the case of the mutants, as compared to the wild-type enzyme. The small observable shifts between the sequencing gel and the cleavage products are due to the unphosphorylated primers that were used in the sequencing reactions.

On the 5'-GGATG-3' strand, both mutants cut the DNA 10 nucleotides away from the site while on the 5'-CATCC-3' strand they cut 14 nucleotides away from the recognition site. These appear to be the major cut sites for both the mutants. A small amount of cleavage similar to the wild-type enzyme was is also observed.

The cleavage pattern of the 256 bp fragment is shown in Figure 14B. The pattern of cleavage is shown in Figure 14B. The pattern of cleavage is similar to the 100 bp fragment. Some cleavage is seen 15 nucleotides away from the recognition site on the 5'-CATCC-3' strand in the case of the mutants. The multiple cut sites for the mutant enzymes could be attributed to the presence of different conformations in these proteins. Or due to the increased flexibility of the spacer region between the two domains. Depending on the DNA substrate, some variation in the intensity of cleavage at these sites was observed. This may be due to the nucleotide sequence around these cut sites. Naturally occurring Type ITS enzymes with multiple cut sites have been reported (Szybalski et al., Gene 100:13-26, 1991).

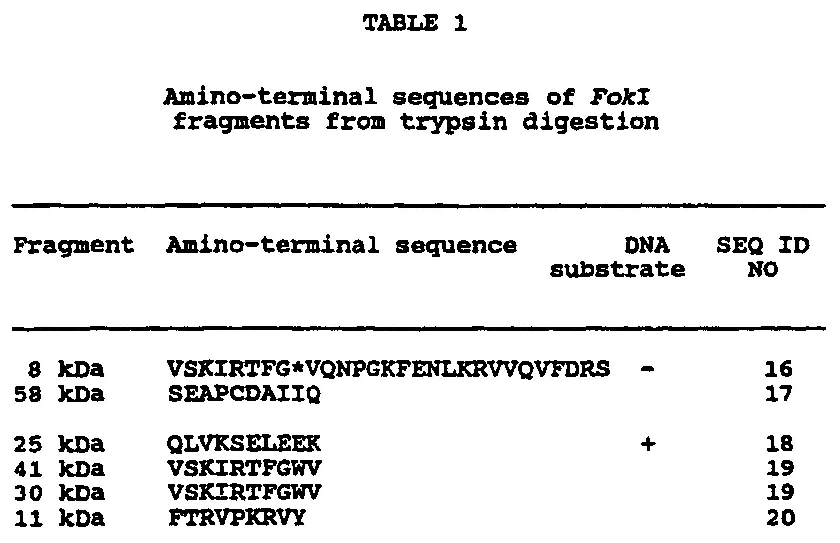

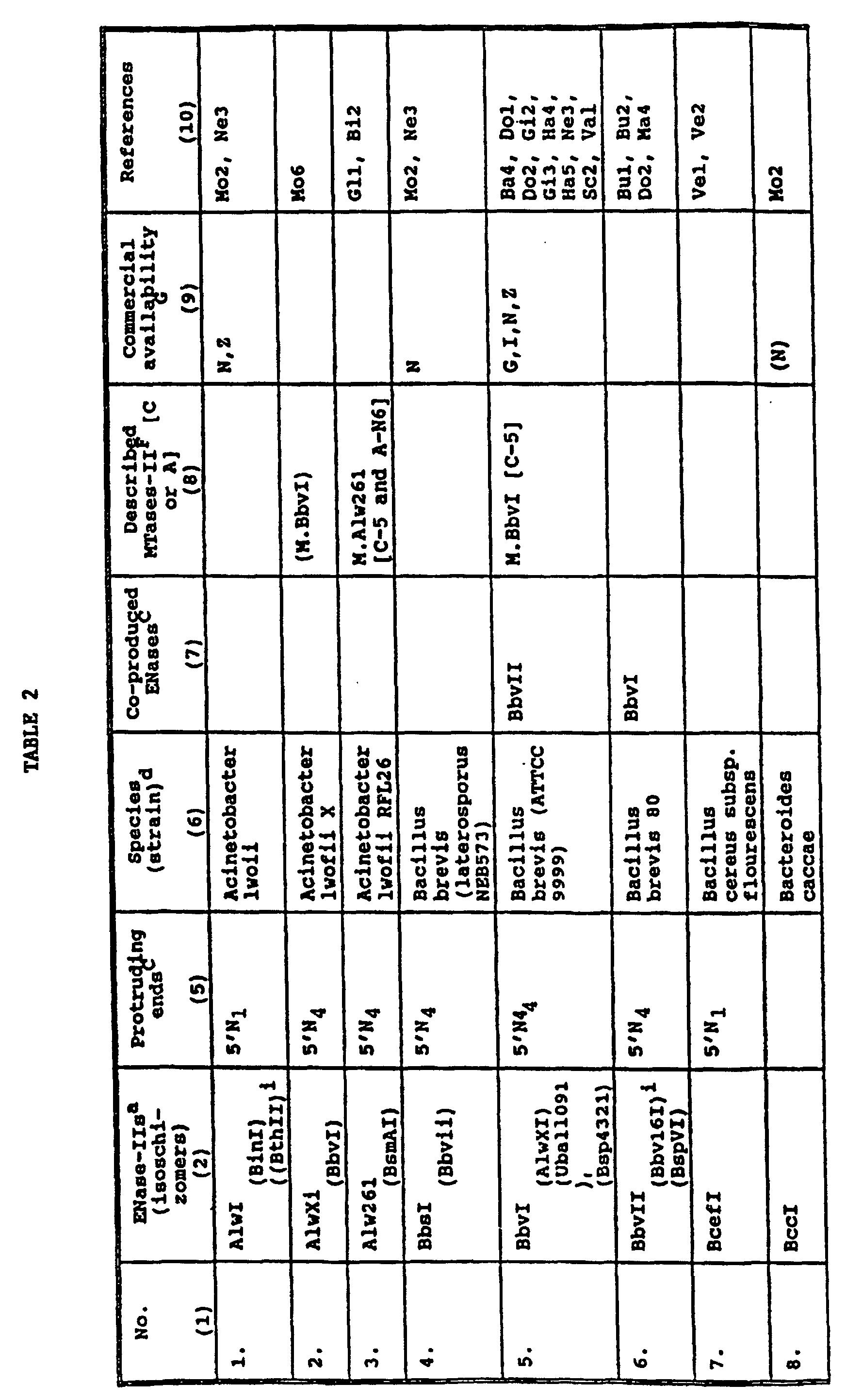

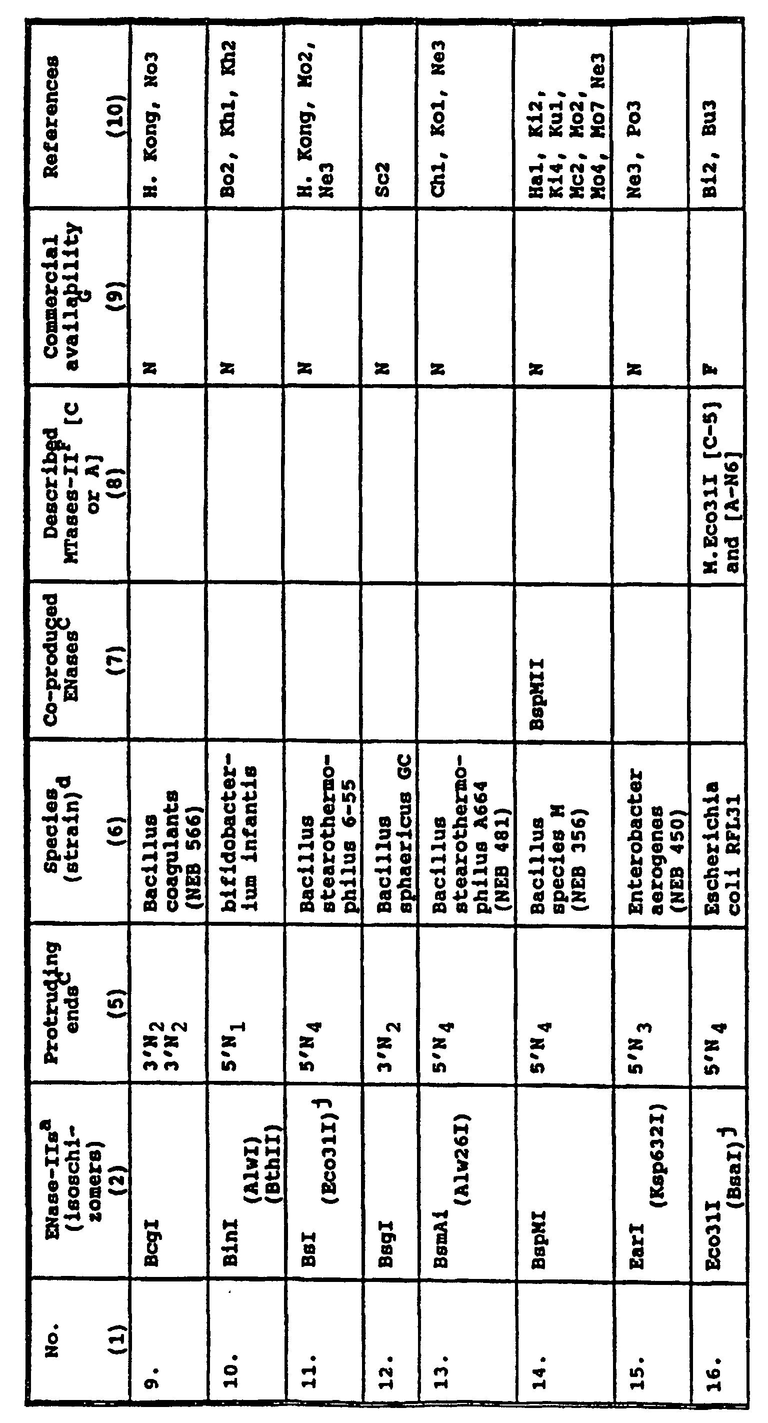

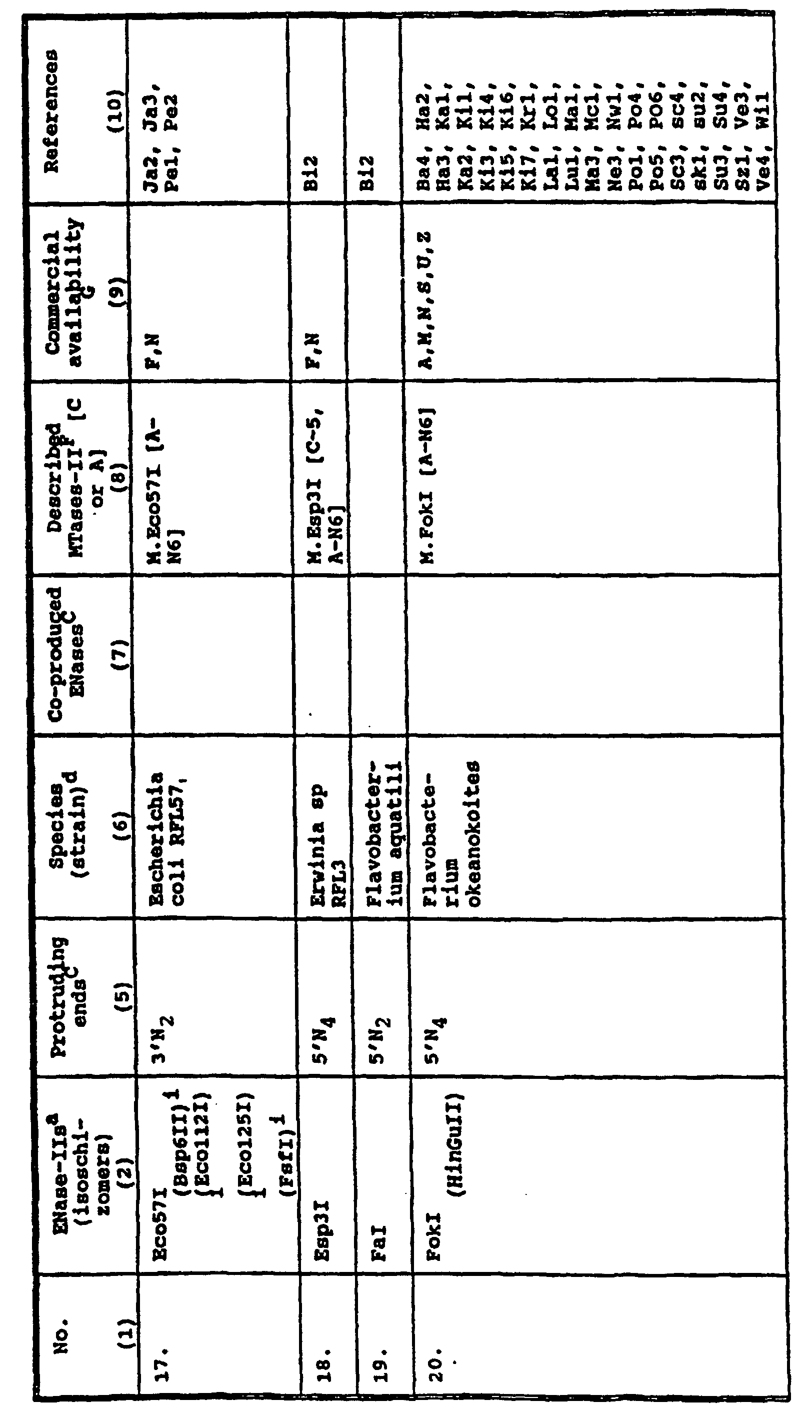

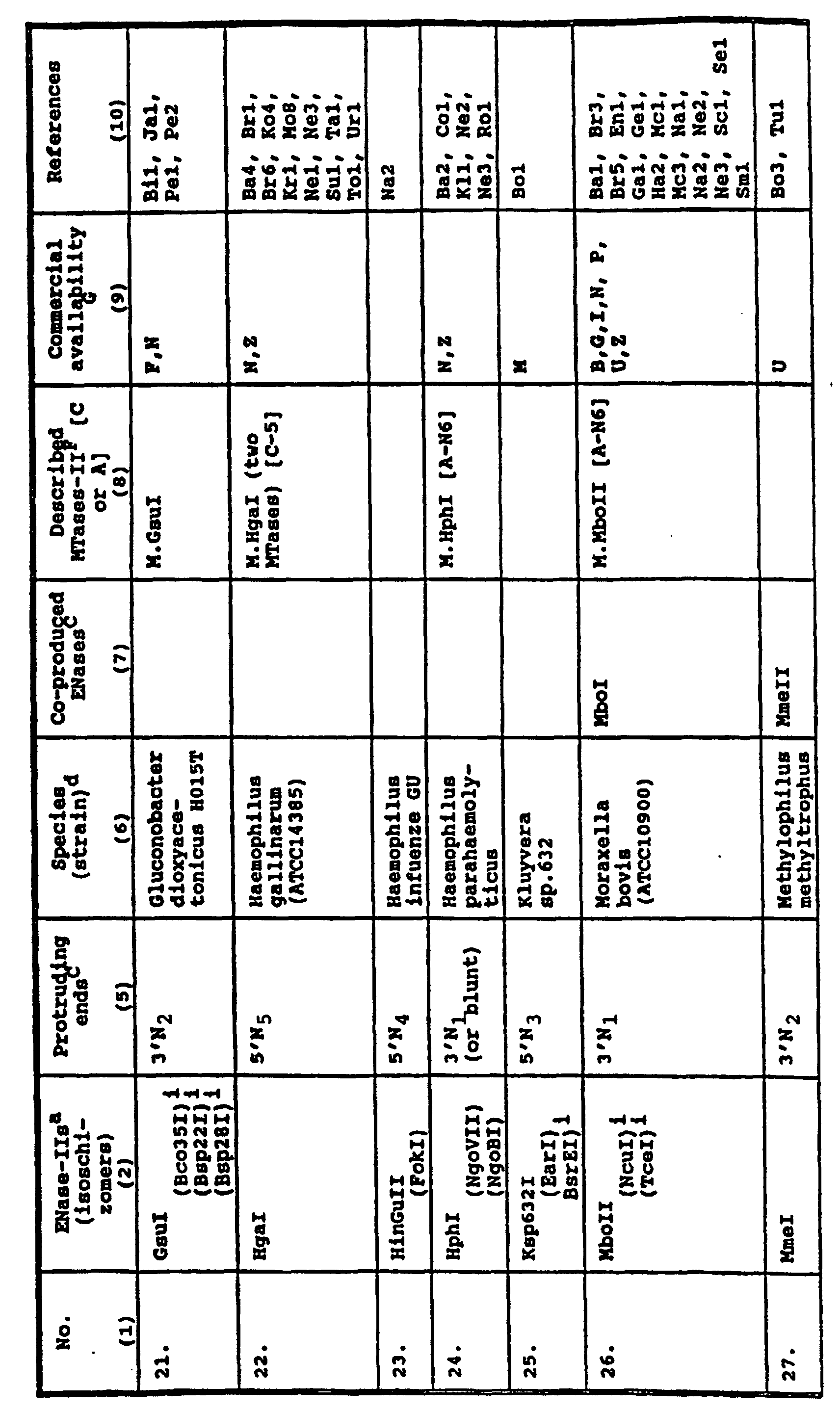

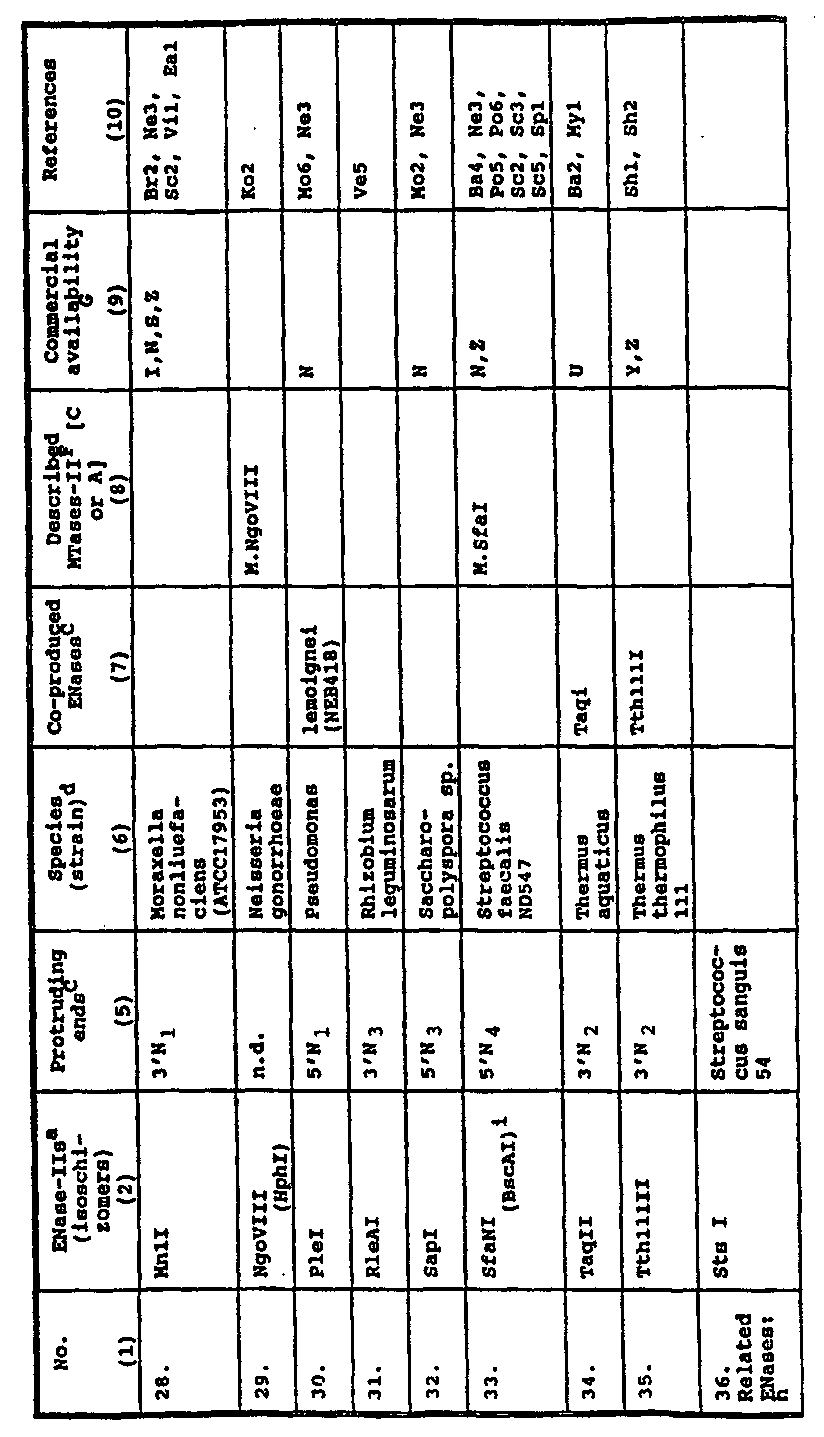

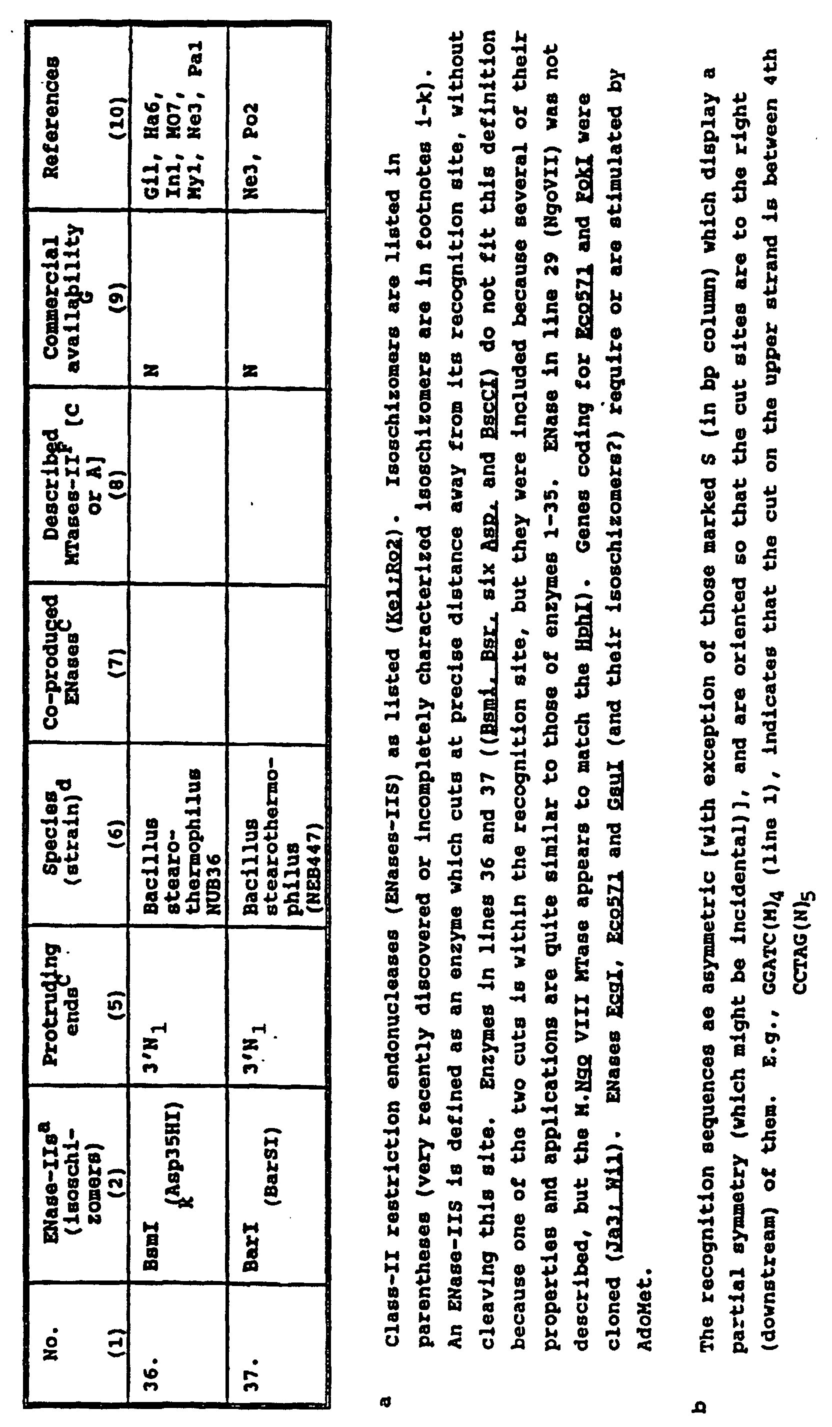

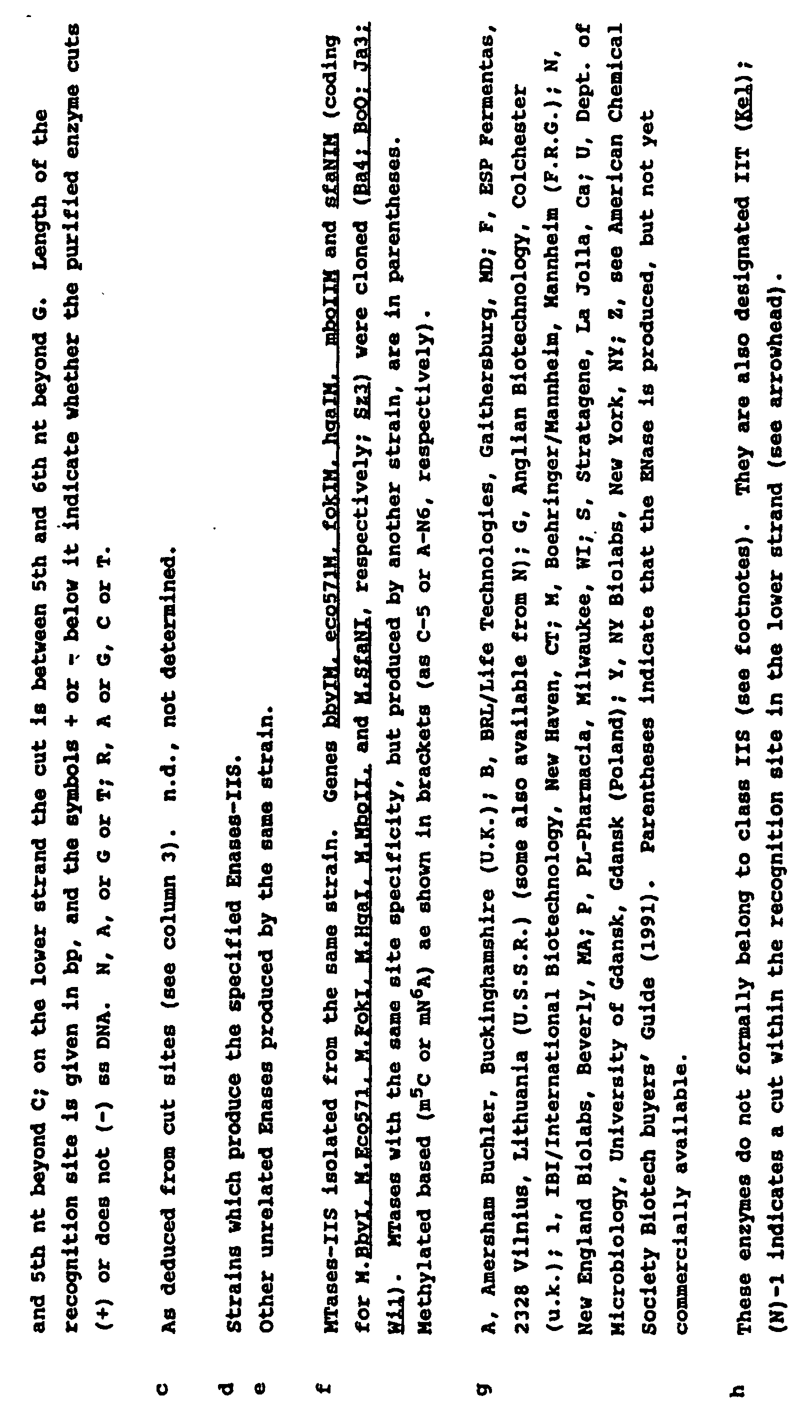

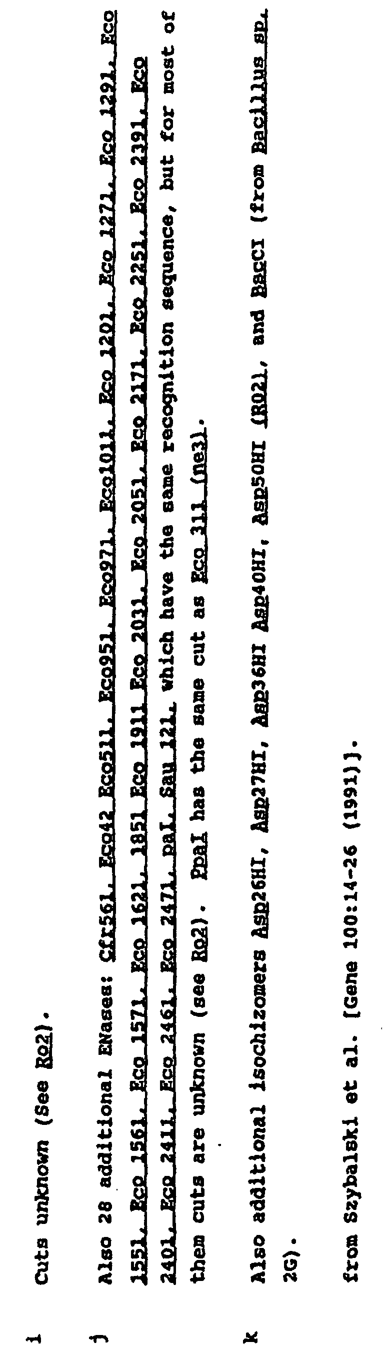

## Claims

1. A DNA construct comprising:
(i) a first DNA segment encoding the catalytic domain of a Type IIS endonuclease which contains the cleavage activity of said Type IIS endonuclease;
(ii) a second DNA segment encoding a sequence-specific recognition domain other than the recognition domain of said Type IIS endonuclease;
(iii) a third DNA segment comprising one or more codons, wherein said third DNA segment is inserted between said first DNA segment and said second DNA segment; and
(iv)a vector
wherein said first DNA segment, said second DNA segment and said third DNA segment are operably linked to said vector so that a single protein is produced.

2. The DNA construct according to claim 1 wherein said Type IIS endonuclease is *Fok*I restriction endonuclease.

3. The DNA construct according to claim 2 wherein said third DNA segment comprises four codons.

4. The DNA construct according to claim 3 wherein said four codons of said third DNA segment are inserted at nucleotide 1152 of the gene encoding said endonuclease.

5. The DNA construct according to claim 2 wherein said third DNA segment comprises 7 codons.

6. The DNA construct according to claim 5 wherein said 7 codons of said third DNA segment are inserted at nucleotide 1152 of the gene encoding said endonuclease.

7. The DNA construct according to claim 2 wherein said recognition domain is selected from the group consisting of: zinc finger motifs, homeo domain motifs, DNA binding domains of repressors, DNA binding domains of oncogenes and naturally occurring sequence-specific DNA binding proteins that recognise >6 base pairs.

8. A procaryotic cell comprising:
(i) a first DNA segment encoding the catalytic domain of a Type IIS endonuclease which contains the cleavage activity of said Type IIS endonuclease;
(ii) a second DNA segment encoding a sequence-specific recognition domain other than the recognition domain of said Type IIS endonuclease;
(iii) a third DNA segment comprising one or more codons, wherein said third DNA segment is inserted between first DNA segment and said second DNA segment; and
(iv)a vector
wherein said first DNA segment, said second DNA segment, and said third DNA segment are operably linked to said vector so that a single protein is produced.

9. The procaryotic cell of claim 8 wherein said third DNA segment comprises four codons.

10. The prokaryotic cell of claim 8 wherein said third DNA segment comprises seven codons.

11. An isolated hybrid Type IIS endonuclease produced by the procaryotic cell of claim 8.

## Patentansprüche

1. Ein DNA-Konstrukt, welches umfaßt:
(i) ein erstes DNA-Segment, welches für die katalytische Domäne einer Typ IIS-Endonuklease codiert, welche die Spaltaktivität dieser Typ IIS-Endonuklease enthält;
(ii) ein zweites DNA-Segment, welches eine sequenzspezifische Erkennungsdomäne außer der Erkennungsdomäne dieser Typ IIS-Endonuklease enthält;
(iii) ein drittes DNA-Segment, welches oder mehrere Codons umfaßt, wobei das dritte DNA-Segment zwischen dem ersten DNA-Segment und dem zweiten DNA-Segment inseriert ist; und
(iv) einen Vektor, wobei das erste DNA-Segment, das zweite DNA-Segment und das dritte DNA-Segment operabel mit diesem Vektor verbunden sind, so daß ein einziges Protein produziert wird.

2. Das DNA-Konstrukt nach Anspruch 1, wobei die Typ IIS-Endonuklease *Fok*I-Restriktionsendonuklease ist.

3. Das DNA-Konstrukt nach Anspruch 2, wobei das dritte DNA-Segment vier Codons umfaßt.

4. Das DNA-Konstrukt nach Anspruch 3, wobei diese vier Codons des dritten DNA-Segmentes bei Nukleotid 1152 des Gens, das für die Endonuklease codiert, inseriert sind.

5. Das DNA-Konstrukt nach Anspruch 2, wobei das dritte DNA-Segment 7 Codons umfaßt.

6. Das DNA-Konstrukt nach Anspruch 5, wobei die 7 Codons des dritten DNA-Segments bei Nukleotid 1152 des Gens inseriert sind, das für die Endonuklease codiert.

7. Das DNA-Konstrukt nach Anspruch 2, wobei die Erkennungsdomäne ausgewählt ist aus der Gruppe bestehend aus: Zinkfingermotiven, Homöodomänenmotiven, DNA-Bindedomänen von Repressoren, DNA-Bindedomänen von Onkogenen und natürlich auftretenden sequenzspezifischen DNA-Bindeproteinen, die >6 Basenpaare erkennen.

8. Eine prokaryotische Zelle, welche umfaßt:
(i) ein erstes DNA-Segment, das für die katalytische Domäne einer Typ IIS-Endonuklease codiert, welche die Spaltaktivität dieser Typ IIS-Endonuklease enthält;
(ii) ein zweites DNA-Segment, das für eine sequenzspezifische Erkennungsdomäne außer der Erkennungsdomäne dieser Typ IIS-Endonuklease codiert;
(iii) ein drittes DNA-Segment, welches ein oder mehrere Codons umfaßt, wobei das dritte DNA-Segment zwischen dem ersten DNA-Segment und dem zweiten DNA-Segment inseriert ist; und
(iv) einen Vektor, wobei ein erste DNA-Segment, das zweite DNA-Segment und das dritte DNA-Segment operabel mit dem Vektor verbunden sind, so daß ein einziges Protein produziert wird.

9. Die prokaryotische Zelle nach Anspruch 8, wobei das dritte DNA-Segment vier Codons umfaßt.

10. Die prokaryotische Zelle nach Anspruch 8, wobei das dritte DNA-Segment sieben Codons umfaßt.

11. Eine isolierte Hybrid-Typ-IIS-Endonuklease, die durch die prokaryotische Zelle nach Anspruch 8 produziert wird.

## Revendications

1. Un assemblage ADN comprenant :
(i) un premier segment d'ADN codant pour le domaine catalytique d'une endonucléase de type IIS, qui contient l'activité de clivage de ladite endonucléase de type IIS ;
(ii) un deuxième segment d'ADN codant pour un domaine de reconnaissance séquence-spécifique autre que le domaine de reconnaissance de ladite endonucléase de type IIS ;
(iii) un troisième segment d'ADN comprenant un ou plusieurs codons, ledit troisième segment d'ADN étant inséré entre ledit premier segment d'ADN et ledit deuxième segment d'ADN ; et
(iv) un vecteur
dans lequel ledit premier segment d'ADN, ledit deuxième segment d'ADN et ledit troisième segment d'ADN sont fonctionnellement liés audit vecteur de manière à ce qu'une seule protéine soit produite.

2. Assemblage ADN selon la revendication 1, dans lequel ladite endonucléase de type IIS est l'endonucléase de restriction *Fok*I.

3. Assemblage ADN selon la revendication 2, dans lequel ledit troisième segment d'ADN comprend quatre codons.

4. Assemblage ADN selon la revendication 3, dans lequel lesdits quatre codons dudit troisième segment d'ADN sont insérés au niveau du nucléotide 1152 du gène codant pour ladite endonucléase.

5. Assemblage ADN selon la revendication 2, dans lequel ledit troisième segment d'ADN comprend 7 codons.

6. Assemblage ADN selon la revendication 5, dans lequel lesdits 7 codons dudit troisième segment d'ADN sont insérés au niveau du nucléotide 1152 du gène codant pour ladite endonucléase.

7. Assemblage ADN selon la revendication 2, dans lequel ledit domaine de reconnaissance est sélectionné dans le groupe consistant en : motifs doigts de zinc, motifs de domaines homéo, domaines de liaison à l'ADN de répresseurs, domaines de liaison à l'ADN d'oncogènes et protéines naturelles de liaison à l'ADN séquence-spécifiques reconnaissant > 6 paires de bases.

8. Cellule procaryote comprenant :
(i) un premier segment d'ADN codant pour le domaine catalytique d'une endonucléase de type IIS, qui contient l'activité de clivage de ladite endonucléase de type IIS ;
(ii) un deuxième segment d'ADN codant pour un domaine de reconnaissance séquence-spécifique autre que le domaine de reconnaissance de ladite endonucléase de type IIS;
(iii) un troisième segment d'ADN comprenant un ou plusieurs codons, ledit troisième segment d'ADN étant inséré entre le premier segment d'ADN et ledit deuxième segment d'ADN ; et
(iv) un vecteur
dans laquelle ledit premier segment d'ADN, ledit deuxième segment d'ADN et ledit troisième segment d'ADN sont fonctionnellement liés audit vecteur de manière à ce qu'une seule protéine soit produite.

9. Cellule procaryote selon la revendication 8, dans laquelle ledit troisième segment d'ADN comprend quatre codons.

10. Cellule procaryote selon la revendication 8, dans laquelle ledit troisième segment d'ADN comprend sept codons.

11. Endonucléase de type IIS hybride isolée produite par la cellule procaryote selon la revendication 8.


### SEQUENCE LISTING

(1) GENERAL INFORMATION:
   (i) APPLICANT: Chandrasegaran, Srinivasan
   (ii) TITLE OF INVENTION: Functional Domains in FokI Restriction Endonuclease
   (iii) NUMBER OF SEQUENCES: 40
   (iv) CORRESPONDENCE ADDRESS:
      (A) ADDRESSEE: Cushman, Darby & Cushman
      (B) STREET: 1100 New York Ave., N.W.
      (C) CITY: Washington
      (D) STATE: D.C.
      (E) COUNTRY: USA
      (F) ZIP: 20005-3918
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Kokulis, Paul N.
      (B) REGISTRATION NUMBER: 16,773
      (C) REFERENCE/DOCKET NUMBER: PNK/4130/122364/CLB
   (ix) TELECOMMUNICATION INFORMATION:
      (A) TELEPHONE: 202-861-3503
      (B) TELEFAX: 202-822-0944
      (C) TELEX: 6714627 CUSH
(2) INFORMATION FOR SEQ ID NO:1:
   (i) SEQUENCE CHARACTERISTICS:
      (A) LENGTH: 5 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1: GGATG
(2) INFORMATION FOR SEQ ID NO:2
   (i) SEQUENCE CHARACTERISTICS:
      (A) LENGTH: 5 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2: CCTAC
(2) INFORMATION FOR SEQ ID NO:3:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 18..35
   (xi) SEQUENCE DESCRIPTION: SEQ ID NO:3:
   CCATGGAGGT TTAAAAT ATG AGA TTT ATT GGC AGC                35
                      Met Arg Phe Ile Gly Ser
                       1               5
(2) INFORMATION FOR SEQ ID NO:4:
   (i) SEQUENCE CHARACTERISTICS:
      (A) LENGTH: 6 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Arg Phe Ile Gly Ser
    1               5
(2) INFORMATION FOR SEQ ID NO:5:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
   ATACCATGGG AATTAAATGA CACAGCATCA                                    30
(2) INFORMATION FOR SEQ ID NO:6:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 22..42
   (xi) SEQUENCE DESCRIPTION: SEQ ID NO:6:
(2) INFORMATION FOR SEQ ID NO:7:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:7;
(2) INFORMATION FOR SEQ ID NO:8
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:8:
   TAGGATCCTC ATTAAAAGTT TATCTCGCCG TTATT 35
(2) INFORMATION FOR SEQ ID NO:9:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:9:
   Asn Asn Gly Glu Ile Asn Phe
   1 5
(2) INFORMATION FOR SEQ ID NO:10:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:10:
   CCTCTGGATG CTCTC 15
(2) INFORMATION FOR SEQ ID NO:11:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:11:
   GAGAGCATCC AGAGG 15
(2) INFORMATION FOR SEQ ID NO:12:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:12:
   TAATTGATTC TTAA 14
(2) INFORMATION FOR SEQ ID NO:13:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:13:
   ATTAAGAATC AATT 14
(2) INFORMATION FOR SEQ ID NO:14:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:14:
   CCTCTGGATG CTCTCAAAAA AAAAAAAAAA 30
(2) INFORMATION FOR SEQ ID NO:15:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:15:
   GAGAGCATCC AGAGGAAAAA AAAAAAAAAA                                30
(2) INFORMATION FOR SEQ ID NO:16:
   (i) SEQUENCE CHARACTERISTICS:
      (A) LENGTH: 29 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:16:
   Val Ser Lys Ile Arg Thr Phe Gly Xaa Val Gln Asn Pro Gly
    1              5                   10
   Lys Phe Glu Asn Leu Lys Arg Val Val Gln Val Phe Asp Arg
   15                  20                  25
   Ser
(2) INFORMATION FOR SEQ ID NO:17:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:17:
   Ser Glu Ala Pro Cys Asp Ala Ile Ile Gln
   1               5                   10
(2) INFORMATION FOR SEQ ID NO:18:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:18:
   Gln Leu Val Lys Ser Glu Leu Glu Glu Lys
   1               5                   10
(2) INFORMATION FOR SEQ ID NO:19:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:19:
   Val Ser Lys Ile Arg Thr Phe Gly Trp Val
   1               5                   10
(2) INFORMATION FOR SEQ ID NO:20:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:20:
   Phe Thr Arg Val Pro Lys Arg Val Tyr
   1               5
(2) INFORMATION FOR SEQ ID NO:21:
   (i) SEQUENCE CHARACTERISTICS:
      (A) LENGTH: 3 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:21:
   Glu Glu Lys
   1
(2) INFORMATION FOR SEQ ID NO:22:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:22:
   Lys Ser Glu Leu
   1
(2) INFORMATION FOR SEQ ID NO:23:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:23:
   Lys Ser Glu Leu Glu Glu Lys
   1 5
(2) INFORMATION FOR SEQ ID NO:24:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:24:
   TAGCAACTAA TTCTTTTTGG ATCTT 25
(2) INFORMATION FOR SEQ ID NO:25:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:25:
   CCATCGATAT AGCCTTTTTT ATT 23
(2) INFORMATION FOR SEQ ID NO:26:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:26:
   GCTCTAGAGG ATCCGGAGGT 20
(2) INFORMATION FOR SEQ ID NO:27
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:27:
   CGCAGTGTTA TCACTCAT 18
(2) INFORMATION FOR SEQ ID NO:28:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:28:
   CTTGGTTGAG TACTCACC 18
(2) INFORMATION FOR SEQ ID NO:29:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:29:
   ACCGAGCTCG AATTCACT 18
(2) INFORMATION FOR SEQ ID NO:30:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:30:
   GATTTCGGCC TATTGGTT 18
(2) INFORMATION FOR SEQ ID NO:31:
   (i) SEQUENCE CHARACTERISTICS:
      (A) LENGTH: 579 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:31:
   Met Val Ser Lys Ile Arg Thr Phe Gly Trp Val Gln Asn Pro
    1              5                   10
   Gly Lys Phe Glu Asn Leu Lys Arg Val Val Gln Val Phe Asp
   15                  20                  25
   Arg Asn Ser Lys Val His Asn Glu Val Lys Asn Ile Lys Ile
       30                  35                  40
   Pro Thr Leu Val Lys Glu Ser Lys Ile Gln Lys Glu Leu Val
           45                  50                  55
   Ala Ile Met Asn Gln His Asp Leu Ile Tyr Thr Tyr Lys Glu
               60                  65                  70
   Leu Val Gly Thr Gly Thr Ser Ile Arg Ser Glu Ala Pro Cys
                   75                  80
   Asp Ala Ile Ile Gln Ala Thr Ile Ala Asp Gln Gly Asn Lys
   85                  90                  95
   Lys Gly Tyr Ile Asp Asn Trp Ser Ser Asp Gly Phe Leu Arg
       100                 105                 110
   Trp Ala His Ala Leu Gly Phe Ile Glu Tyr Ile Asn Lys Ser
           115                 120                 125
   Asp Ser Phe Val Ile Thr Asp Val Gly Leu Ala Tyr Ser Lys
               130                 135                 140
   Ser Ala Asp Gly Ser Ala Ile Glu Lys Glu Ile Leu Ile Glu
                   145                 150
   Ala Ile Ser Ser Tyr Pro Pro Ala Ile Arg Ile Leu Thr Leu
   155                 160                 165
   Leu Glu Asp Gly Gln His Leu Thr Lys Phe Asp Leu Gly Lys
       170                 175                 180
   Asn Leu Gly Phe Ser Gly Glu Ser Gly Phe Thr Ser Leu Pro
           185                 190                 195
   Glu Gly Ile Leu Leu Asp Thr Leu Ala Asn Ala Met Pro Lys
               200                 205                 210
   Asp Lys Gly Glu Ile Arg Asn Asn Trp Glu Gly Ser Ser Asp
                   215                 220
   Lys Tyr Ala Arg Met Ile Gly Gly Trp Leu Asp Lys Leu Gly
   225                 230                 235
   Leu Val Lys Gln Gly Lys Lys Glu Phe Ile Ile Pro Thr Leu
       240                 245                 250
   Gly Lys Pro Asp Asn Lys Glu Phe Ile Ser His Ala Phe Lys
           255                 260                 265
   Ile Thr Gly Glu Gly Leu Lys Val Leu Arg Arg Ala Lys Gly
               270                 275                 280
   Ser Thr Lys Phe Thr Arg Val Pro Lys Arg Val Tyr Trp Glu
                   285                 290
   Met Leu Ala Thr Asn Leu Thr Asp Lys Glu Tyr Val Arg Thr
   295                 300                 305
   Arg Arg Ala Leu Ile Leu Glu Ile Leu Ile Lys Ala Gly Ser
       310                 315                 320
   Leu Lys Ile Glu Gln Ile Gln Asp Asn Leu Lys Lys Leu Gly
           325                 330                 335
   Phe Asp Glu Val Ile Glu Thr Ile Glu Asn Asp Ile Lys Gly
               340                 345                 350
   Leu Ile Asn Thr Gly Ile Phe Ile Glu Ile Lys Gly Arg Phe
                   355                 360
   Tyr Gln Leu Lys Asp His Ile Leu Gln Phe Val Ile Pro Asn
   365                 370                 375
   Arg Gly Val Thr Lys Gln Leu Val Lys Ser Glu Leu Glu Glu
       380                 385                 390
   Lys Lys Ser Glu Leu Arg His Lys Leu Lys Tyr Val Pro His
           395                 400                 405
   Glu Tyr Ile Glu Leu Ile Glu Ile Ala Arg Asn Ser Thr Gln
               410                 415                 420
   Asp Arg Ile Leu Glu Met Lys Val Met Glu Phe Phe Met Lys
                   425                 430
   Val Tyr Gly Tyr Arg Gly Lys His Leu Gly Gly Ser Arg Lys
   435                 440                 445
   Pro Asp Gly Ala Ile Tyr Thr Val Gly Ser Pro Ile Asp Tyr
       450                 455                 460
   Gly Val Ile Val Asp Thr Lys Ala Tyr Ser Gly Gly Tyr Asn
           465                 470                 475
   Leu Pro Ile Gly Gln Ala Asp Glu Met Gln Arg Tyr Val Glu
               480                 485                 490
   Glu Asn Gln Thr Arg Asn Lys His Ile Asn Pro Asn Glu Trp
                   495                 500
   Trp Lys Val Tyr Pro Ser Ser Val Thr Glu Phe Lys Phe Leu
   505                 510                 515
   Phe Val Ser Gly His Phe Lys Gly Asn Tyr Lys Ala Gln Leu
       520                 525                 530
   Thr Arg Leu Asn His Ile Thr Asn Cys Asn Gly Ala Val Leu
           535                 540                 545
   Ser Val Glu Glu Leu Leu Ile Gly Gly Glu Met Ile Lys Ala
               550                 555                 560
   Gly Thr Leu Thr Leu Glu Glu Val Arg Arg Lys Phe Asn Asn
                   565                 570
   Gly Glu Ile Asn Phe
   575
(2) INFORMATION FOR SEQ ID NO:32:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:32:
   Lys Gln Leu Val Lys Ser Glu Leu Glu Glu Lys
   1               5                   10
(2) INFORMATION FOR SEQ ID NO:33:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:33:
   AAGCAACTAG TCAAAAGTGA ACTGGAGGAG AAG                33
(2) INFORMATION FOR SEQ ID NO:34:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:34:
   Leu Val Lys Ser Glu Leu Lys Ser Glu Leu Glu Glu Lys
   1               5                   10
(2) INFORMATION FOR SEQ ID NO:35:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:35:
   GGACTAGTCA AATCTGAACT TAAAAGTGAA CTGGAGGAGA AG          42
(2) INFORMATION FOR SEQ ID NO:36:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:36:
   Leu Val Lys Ser Glu Leu Glu Glu Lys Lys Ser Glu Leu Glu
    1              5                   10
   Glu Lys
   15
(2) INFORMATION FOR SEQ ID NO:37:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:37:
   GGACTAGTCA AATCTGAACT TGAGGAGAAG AAAAGTGAAC TGGAGGAGAA G 51
(2) INFORMATION FOR SEQ ID NO:38:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:38:
   Asn Phe Xaa Xaa
   1
(2) INFORMATION FOR SEQ ID NO:39:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:39:
   TTGAAAATTA CTCCTAGGGG CCCCCCT 27
(2) INFORMATION FOR SEQ ID NO:40:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:40:
   GGATGNNNNNNNNNNNNNNNNNN 23